# EUROPEAN PATENT APPLICATION

(11) **EP 2 487 250 A1**
(43) Date of publication of application: **15.08.2012**
(21) Application number: 10821891.8
(22) Date of filing: 28.09.2010
(51) Int. Cl.: C12Q 1/68, C12N 15/09

(54) **METHOD FOR DETECTING FUSION GENE**

(30) Priority: 06.10.2009 JP 2009232473
(71) Applicant: FUJIREBIO INC., Chuo-ku Tokyo 103-0007 (JP)
(72) Inventor: OMI, Kazuya, Tokyo 103-0007 (JP)
(74) Representative: Keirstead, Tanis Evelyne
(86) International application number: PCT/JP2010/066768
(87) International publication number: WO 2011/043220

(57) **Abstract**

Disclosed is a means which enables simple and rapid detection of the presence of any fusion genes including even unknown fusion genes. The method for measuring a fusion gene(s) according to the present invention is applied to a sample separated from a living body, and comprises: measuring expressions of a 5'-region and a 3'-region of one of the component genes of the fusion gene(s) which may be present in the living body, wherein said 5'-region is upstream of and said 3'-region is downstream of a fusion point in said one of the component genes; and comparing the expression of the 5'-region with the expression of the 3'-region. The fusion gene to be measured is e.g. a fusion gene between *ALK* gene and another gene.

SEQUENCE LISTING

<110>  FUJIREBIO INC.

<120>  Method for measurement of fusion gene

<130>  PF420-PCT

<160>  25

<170>  PatentIn version 3.5

<210>  1
<211>  6222
<212>  DNA
<213>  Homo sapiens


<220>
<221>  CDS
<222>  (908)..(5770)

<400>  1
gggggcggca gcggtggtag cagctggtac ctcccgccgc ctctgttcgg agggtcgcgg      60
ggcaccgagg tgctttccgg ccgccctctg gtcggccacc caaagccgcg ggcgctgatg     120
atgggtgagg agggggcggc aagatttcgg gcgcccctgc cctgaacgcc ctcagctgct     180
gccgccgggg ccgctccagt gcctgcgaac tctgaggagc cgaggcgccg gtgagagcaa     240
ggacgctgca aacttgcgca gcgcgggggc tgggattcac gcccagaagt tcagcaggca     300
gacagtccga agccttcccg cagcggagag atagcttgag ggtgcgcaag acggcagcct     360
ccgccctcgg ttcccgccca gaccgggcag aagagcttgg aggagccaaa aggaacgcaa     420
aaggcggcca ggacagcgtg cagcagctgg gagccgccgt tctcagcctt aaaagttgca     480
gagattggag gctgccccga gaggggacag accccagctc cgactgcggg gggcaggaga     540
ggacggtacc caactgccac ctcccttcaa ccatagtagt tcctctgtac cgagcgcagc     600
gagctacaga cgggggcgcg gcactcggcg cggagagcgg gaggctcaag gtcccagcca     660
gtgagcccag tgtgcttgag tgtctctgga ctcgcccctg agcttccagg tctgtttcat     720
ttagactcct gctcgcctcc gtgcagttgg gggaaagcaa gagacttgcg cgcacgcaca     780
gtcctctgga gatcaggtgg aaggagccgc tgggtaccaa ggactgttca gagcctcttc     840
ccatctcggg gagagcgaag ggtgaggctg ggcccggaga gcagtgtaaa cggcctcctc     900
cggcggg atg gga gcc atc ggg ctc ctg tgg ctc ctg ccg ctg ctg ctt      949
tcc acg gca gct gtg ggc tcc ggg atg ggg acc ggc cag cgc gcg ggc      997
tcc cca gct gcg ggg ccg ccg ctg cag ccc cgg gag cca ctc agc tac     1045
tcg cgc ctg cag agg aag agt ctg gca gtt gac ttc gtg gtg ccc tcg     1093
ctc ttc cgt gtc tac gcc cgg gac cta ctg ctg cca cca tcc tcc tcg     1141
gag ctg aag gct ggc agg ccc gag gcc cgc ggc tcg cta gct ctg gac     1189
tgc gcc ccg ctg ctc agg ttg ctg ggg ccg gcg ccg ggg gtc tcc tgg      1237
acc gcc ggt tca cca gcc ccg gca gag gcc cgg acg ctg tcc agg gtg      1285
ctg aag ggc ggc tcc gtg cgc aag ctc cgg cgt gcc aag cag ttg gtg      1333
ctg gag ctg ggc gag gag gcg atc ttg gag ggt tgc gtc ggg ccc ccc      1381
ggg gag gcg gct gtg ggg ctg ctc cag ttc aat ctc agc gag ctg ttc      1429
agt tgg tgg att cgc caa ggc gaa ggg cga ctg agg atc cgc ctg atg      1477
ccc gag aag aag gcg tcg gaa gtg ggc aga gag gga agg ctg tcc gcg      1525
gca att cgc gcc tcc cag ccc cgc ctt ctc ttc cag atc ttc ggg act      1573
ggt cat agc tcc ttg gaa tca cca aca aac atg cct tct cct tct cct      1621
gat tat ttt aca tgg aat ctc acc tgg ata atg aaa gac tcc ttc cct      1669
ttc ctg tct cat cgc agc cga tat ggt ctg gag tgc agc ttt gac ttc      1717
ccc tgt gag ctg gag tat tcc cct cca ctg cat gac ctc agg aac cag      1765
agc tgg tcc tgg cgc cgc atc ccc tcc gag gag gcc tcc cag atg gac      1813
ttg ctg gat ggg cct ggg gca gag cgt tct aag gag atg ccc aga ggc      1861
tcc ttt ctc ctt ctc aac acc tca gct gac tcc aag cac acc atc ctg      1909
agt ccg tgg atg agg agc agc agt gag cac tgc aca ctg gcc gtc tcg      1957
gtg cac agg cac ctg cag ccc tct gga agg tac att gcc cag ctg ctg      2005
ccc cac aac gag gct gca aga gag atc ctc ctg atg ccc act cca ggg      2053
aag cat ggt tgg aca gtg ctc cag gga aga atc ggg cgt cca gac aac      2101
cca ttt cga gtg gcc ctg gaa tac atc tcc agt gga aac cgc agc ttg 2149
tct gca gtg gac ttc ttt gcc ctg aag aac tgc agt gaa gga aca tcc 2197
cca ggc tcc aag atg gcc ctg cag agc tcc ttc act tgt tgg aat ggg 2245
aca gtc ctc cag ctt ggg cag gcc tgt gac ttc cac cag gac tgt gcc 2293
cag gga gaa gat gag agc cag atg tgc cgg aaa ctg cct gtg ggt ttt 2341
tac tgc aac ttt gaa gat ggc ttc tgt ggc tgg acc caa ggc aca ctg 2389
tca ccc cac act cct caa tgg cag gtc agg acc cta aag gat gcc cgg 2437
ttc cag gac cac caa gac cat gct cta ttg ctc agt acc act gat gtc 2485
ccc gct tct gaa agt gct aca gtg acc agt gct acg ttt cct gca ccg 2533
atc aag agc tct cca tgt gag ctc cga atg tcc tgg ctc att cgt gga 2581
gtc ttg agg gga aac gtg tcc ttg gtg cta gtg gag aac aaa acc ggg 2629
aag gag caa ggc agg atg gtc tgg cat gtc gcc gcc tat gaa ggc ttg 2677
agc ctg tgg cag tgg atg gtg ttg cct ctc ctc gat gtg tct gac agg 2725
ttc tgg ctg cag atg gtc gca tgg tgg gga caa gga tcc aga gcc atc 2773
gtg gct ttt gac aat atc tcc atc agc ctg gac tgc tac ctc acc att 2821
agc gga gag gac aag atc ctg cag aat aca gca ccc aaa tca aga aac 2869
ctg ttt gag aga aac cca aac aag gag ctg aaa ccc ggg gaa aat tca 2917
cca aga cag acc ccc atc ttt gac cct aca gtt cat tgg ctg ttc acc 2965
aca tgt ggg gcc agc ggg ccc cat ggc ccc acc cag gca cag tgc aac 3013
aac gcc tac cag aac tcc aac ctg agc gtg gag gtg ggg agc gag ggc 3061
ccc ctg aaa ggc atc cag atc tgg aag gtg cca gcc acc gac acc tac 3109
agc atc tcg ggc tac gga gct gct ggc ggg aaa ggc ggg aag aac acc 3157
atg atg cgg tcc cac ggc gtg tct gtg ctg ggc atc ttc aac ctg gag 3205
aag gat gac atg ctg tac atc ctg gtt ggg cag cag gga gag gac gcc 3253
tgc ccc agt aca aac cag tta atc cag aaa gtc tgc att gga gag aac 3301
aat gtg ata gaa gaa gaa atc cgt gtg aac aga agc gtg cat gag tgg 3349
gca gga ggc gga gga gga ggg ggt gga gcc acc tac gta ttt aag atg 3397
aag gat gga gtg ccg gtg ccc ctg atc att gca gcc gga ggt ggt ggc 3445
agg gcc tac ggg gcc aag aca gac acg ttc cac cca gag aga ctg gag 3493
aat aac tcc tcg gtt cta ggg cta aac ggc aat tcc gga gcc gca ggt 3541
ggt gga ggt ggc tgg aat gat aac act tcc ttg ctc tgg gcc gga aaa 3589
tct ttg cag gag ggt gcc acc gga gga cat tcc tgc ccc cag gcc atg 3637
aag aag tgg ggg tgg gag aca aga ggg ggt ttc gga ggg ggt gga ggg 3685
ggg tgc tcc tca ggt gga gga ggc gga gga tat ata ggc ggc aat gca 3733
gcc tca aac aat gac ccc gaa atg gat ggg gaa gat ggg gtt tcc ttc 3781
atc agt cca ctg ggc atc ctg tac acc cca gct tta aaa gtg atg gaa 3829
ggc cac ggg gaa gtg aat att aag cat tat cta aac tgc agt cac tgt 3877
gag gta gac gaa tgt cac atg gac cct gaa agc cac aag gtc atc tgc 3925
ttc tgt gac cac ggg acg gtg ctg gct gag gat ggc gtc tcc tgc 3970
att gtg tca ccc acc ccg gag cca cac ctg cca ctc tcg ctg atc 4015
ctc tct gtg gtg acc tct gcc ctc gtg gcc gcc ctg gtc ctg gct 4060
ttc tcc ggc atc atg att gtg tac cgc cgg aag cac cag gag ctg 4105
caa gcc atg cag atg gag ctg cag agc cct gag tac aag ctg agc 4150
aag ctc cgc acc tcg acc atc atg acc gac tac aac ccc aac tac 4195
tgc ttt gct ggc aag acc tcc tcc atc agt gac ctg aag gag gtg 4240
ccg cgg aaa aac atc acc ctc att cgg ggt ctg ggc cat ggc gcc 4285
ttt ggg gag gtg tat gaa ggc cag gtg tcc gga atg ccc aac gac 4330
cca agc ccc ctg caa gtg gct gtg aag acg ctg cct gaa gtg tgc 4375
tct gaa cag gac gaa ctg gat ttc ctc atg gaa gcc ctg atc atc 4420
agc aaa ttc aac cac cag aac att gtt cgc tgc att ggg gtg agc 4465
ctg caa tcc ctg ccc cgg ttc atc ctg ctg gag ctc atg gcg ggg 4510
gga gac ctc aag tcc ttc ctc cga gag acc cgc cct cgc ccg agc 4555
cag ccc tcc tcc ctg gcc atg ctg gac ctt ctg cac gtg gct cgg 4600
gac att gcc tgt ggc tgt cag tat ttg gag gaa aac cac ttc atc 4645
cac cga gac att gct gcc aga aac tgc ctc ttg acc tgt cca ggc 4690
cct gga aga gtg gcc aag att gga gac ttc ggg atg gcc cga gac 4735
atc tac agg gcg  agc tac tat aga aag  gga ggc tgt gcc atg  ctg        4780
cca gtt aag tgg  atg ccc cca gag gcc  ttc atg gaa gga ata  ttc        4825
act tct aaa aca  gac aca tgg tcc ttt  gga gtg ctg cta tgg  gaa        4870
atc ttt tct ctt  gga tat atg cca tac  ccc agc aaa agc aac  cag        4915
gaa gtt ctg gag  ttt gtc acc agt gga  ggc cgg atg gac cca  ccc        4960
aag aac tgc cct  ggg cct gta tac cgg  ata atg act cag tgc  tgg        5005
caa cat cag cct  gaa gac agg ccc aac  ttt gcc atc att ttg  gag        5050
agg att gaa tac  tgc acc cag gac ccg  gat gta atc aac acc  gct        5095
ttg ccg ata gaa  tat ggt cca ctt gtg  gaa gag gaa gag aaa  gtg        5140
cct gtg agg ccc  aag gac cct gag ggg  gtt cct cct ctc ctg  gtc        5185
tct caa cag gca  aaa cgg gag gag gag  cgc agc cca gct gcc  cca        5230
cca cct ctg cct  acc acc tcc tct ggc  aag gct gca aag aaa  ccc        5275
aca gct gca gag  atc tct gtt cga gtc  cct aga ggg ccg gcc  gtg        5320
gaa ggg gga cac  gtg aat atg gca ttc  tct cag tcc aac cct  cct        5365
tcg gag ttg cac  aag gtc cac gga tcc  aga aac aag ccc acc  agc        5410
ttg tgg aac cca  acg tac ggc tcc tgg  ttt aca gag aaa ccc  acc        5455
aaa aag aat aat  cct ata gca aag aag  gag cca cac gac agg  ggt        5500
aac ctg ggg ctg  gag gga agc tgt act  gtc cca cct aac gtt  gca        5545
act ggg aga ctt  ccg ggg gcc tca ctg  ctc cta gag ccc tct  tcg        5590
ctg act gcc aat atg aag gag gta cct ctg ttc agg cta cgt cac 5635
ttc cct tgt ggg aat gtc aat tac ggc tac cag caa cag ggc ttg 5680
ccc tta gaa gcc gct act gcc cct gga gct ggt cat tac gag gat 5725
acc att ctg aaa agc aag aat agc atg aac cag cct ggg ccc tga 5770
gctcggtcgc acactcactt ctcttccttg ggatccctaa gaccgtggag gagagagagg 5830
caatggctcc ttcacaaacc agagaccaaa tgtcacgttt tgttttgtgc caacctattt 5890
tgaagtacca ccaaaaaagc tgtattttga aaatgcttta gaaaggtttt gagcatgggt 5950
tcatcctatt ctttcgaaag aagaaaatat cataaaaatg agtgataaat acaaggccca 6010
gatgtggttg cataaggttt ttatgcatgt ttgttgtata cttccttatg cttctttcaa 6070
attgtgtgtg ctctgcttca atgtagtcag aattagctgc ttctatgttt catagttggg 6130
gtcatagatg tttccttgcc ttgttgatgt ggacatgagc catttgaggg gagagggaac 6190
ggaaataaag gagttatttg taatgactaa aa 6222

<210> 2
<211> 1620
<212> PRT
<213> Homo sapiens

<400> 2

<210> 3
<211> 20
<212> DNA
<213> Artificial Sequence

<220>
<223> primer ALK_F_2486, for amplification of ALK 5' region

<400> 3
cccgcttctg aaagtgctac 20

<210> 4
<211> 20
<212> DNA
<213> Artificial Sequence

<220>
<223> primer ALK_R_2629, for amplification of ALK 5' region

<400> 4
cccggttttg ttctccacta 20

<210> 5
<211> 20
<212> DNA
<213> Artificial Sequence

<220>
<223> primer ALK_F_4801, for amplification of ALK 3' region

<400> 5
agaggccttc atggaaggaa 20

<210> 6
<211> 20
<212> DNA
<213> Artificial Sequence

<220>
<223> primer ALK_R_4865, for amplification of ALK 3' region

<400> 6
atagcagcac tccaaaggac 20

<210> 7
<211> 21
<212> DNA
<213> Artificial Sequence

<220>
<223> primer TBP_F_115, for measurement of housekeeping gene TBP

<400> 7
ccaaggaatt gaggaagttg c 21

<210> 8
<211> 20
<212> DNA
<213> Artificial Sequence

<220>

<223>  primer TBP_R_339, for measurement of housekeeping gene TBP

<400>  8
gtgccataag gcatcattgg                                                    20

<210>  9
<211>  3926
<212>  DNA
<213>  Homo sapiens

<400>  9
ggcggcgcgg cgcggcgctc gcggctgctg cctgggaggg aggccgggca ggcggctgag      60

cggcgcggct ctcaacgtga cggggaagtg gttcgggcgg ccgcggctta ctaccccagg     120

gcgaacggac ggacgacgga ggcgggagcc ggtagccgag ccgggcgacc tagagaacga     180

gcgggtcagg ctcagcgtcg gccactctgt cggtccgctg aatgaagtgc ccgcccctct     240

gagcccggag cccggcgctt tccccgcaag atggacggtt tcgccggcag tctcgatgat     300

agtatttctg ctgcaagtac ttctgatgtt caagatcgcc tgtcagctct tgagtcacga     360

gttcagcaac aagaagatga aatcactgtg ctaaaggcgg ctttggctga tgttttgagg     420

cgtcttgcaa tctctgaaga tcatgtggcc tcagtgaaaa aatcagtctc aagtaaaggc     480

caaccaagcc ctcgagcagt tattcccatg tcctgtataa ccaatggaag tggtgcaaac     540

agaaaaccaa gtcataccag tgctgtctca attgcaggaa aagaaactct ttcatctgct     600

gctaaaagtg gtacagaaaa aaagaaagaa aaaccacaag gacagagaga aaaaaaagag     660

gaatctcatt ctaatgatca aagtccacaa attcgagcat caccttctcc ccagccctct     720

tcacaacctc tccaaataca cagacaaact ccagaaagca agaatgctac tcccaccaaa     780

agcataaaac gaccatcacc agctgaaaag tcacataatt cttgggaaaa ttcagatgat     840

agccgtaata aattgtcgaa aataccttca acacccaaat taataccaaa agttaccaaa     900

actgcagaca agcataaaga tgtcatcatc aaccaagaag gagaatatat taaaatgttt     960

atgcgcggtc ggccaattac catgttcatt ccttccgatg ttgacaacta tgatgacatc    1020

agaacggaac tgcctcctga gaagctcaaa ctggagtggg catatggtta tcgaggaaag    1080

gactgtagag ctaatgttta ccttcttccg accggggaaa tagtttattt cattgcatca    1140

gtagtagtac tatttaatta tgaggagaga actcagcgac actacctggg ccatacagac    1200

tgtgtgaaat gccttgctat acatcctgac aaaattagga ttgcaactgg acagatagct    1260

ggcgtggata aagatggaag gcctctacaa ccccacgtca gagtgtggga ttctgttact    1320

ctatccacac tgcagattat tggacttggc acttttgagc gtggagtagg atgcctggat    1380

ttttcaaaag cagattcagg tgttcattta tgtgttattg atgactccaa tgagcatatg    1440

cttactgtat gggactggca gaagaaagca aaaggagcag aaataaagac aacaaatgaa    1500

gttgttttgg ctgtggagtt tcacccaaca gatgcaaata ccataattac atgcggtaaa    1560

tctcatattt tcttctggac ctggagcggc aattcactaa caagaaaaca gggaattttt    1620

gggaaatatg aaaagccaaa atttgtgcag tgtttagcat tcttggggaa tggagatgtt    1680

cttactggag actcaggtgg agtcatgctt atatggagca aaactactgt agagcccaca    1740

cctgggaaag gacctaaagt gtaccgccgg aagcaccagg agctgcaagc catgcagatg    1800

gagctgcaga gccctgagta caagctgagc aagctccgca cctcgaccat catgaccgac    1860

tacaacccca actactgctt tgctggcaag acctcctcca tcagtgacct gaaggaggtg    1920

ccgcggaaaa acatcaccct cattcggggt ctgggccatg gagcctttgg ggaggtgtat 1980
gaaggccagg tgtccggaat gcccaacgac ccaagccccc tgcaagtggc tgtgaagacg 2040
ctgcctgaag tgtgctctga acaggacgaa ctggatttcc tcatggaagc cctgatcatc 2100
agcaaattca accaccagaa cattgttcgc tgcattgggg tgagcctgca atccctgccc 2160
cggttcatcc tgctggagct catggcgggg ggagacctca agtccttcct ccgagagacc 2220
cgccctcgcc cgagccagcc ctcctccctg gccatgctgg accttctgca cgtggctcgg 2280
gacattgcct gtggctgtca gtatttggag gaaaaccact tcatccaccg agacattgct 2340
gccagaaact gcctcttgac ctgtccaggc cctggaagag tggccaagat tggagacttc 2400
gggatggccc gagacatcta cagggcgagc tactatagaa agggaggctg tgccatgctg 2460
ccagttaagt ggatgccccc agaggccttc atggaaggaa tattcacttc taaaacagac 2520
acatggtcct ttggagtgct gctatgggaa atcttttctc ttggatatat gccatacccc 2580
agcaaaagca accaggaagt tctggagttt gtcaccagtg gaggccggat ggacccaccc 2640
aagaactgcc ctgggcctgt ataccggata atgactcagt gctggcaaca tcagcctgaa 2700
gacaggccca actttgccat cattttggag aggattgaat actgcaccca ggacccggat 2760
gtaatcaaca ccgctttgcc gatagaatat ggtccacttg tggaagagga agagaaagtg 2820
cctgtgaggc ccaaggaccc tgagggggtt cctcctctcc tggtctctca acaggcaaaa 2880
cgggaggagg agcgcagccc agctgcccca ccacctctgc ctaccacctc ctctggcaag 2940
gctgcaaaga aacccacagc tgcagaggtc tctgttcgag tccctagagg gccggccgtg 3000
gaagggggac acgtgaatat ggcattctct cagtccaacc ctccttcgga gttgcacagg 3060
gtccacggat ccagaaacaa gcccaccagc ttgtggaacc caacgtacgg ctcctggttt 3120
acagagaaac ccaccaaaaa gaataatcct atagcaaaga aggagccaca cgagaggggt 3180
aacctggggc tggagggaag ctgtactgtc ccacctaacg ttgcaactgg gagacttccg 3240
ggggcctcac tgctcctaga gccctcttcg ctgactgcca atatgaagga ggtacctctg 3300
ttcaggctac gtcacttccc ttgtgggaat gtcaattacg gctaccagca acagggcttg 3360
cccttagaag ccgctactgc ccctggagct ggtcattacg aggataccat tctgaaaagc 3420
aagaatagca tgaaccagcc tgggccctga gctcggtcac acactcactt ctcttccttg 3480
ggatccctaa gaccgtggag gagagagagg caatcaatgg ctccttcaca aaccagagac 3540
caaatgtcac gttttgtttt gtgccaacct attttgaagt accaccaaaa aagctgtatt 3600
ttgaaaatgc tttagaaagg ttttgagcat gggttcatcc tattctttcg aaagaagaaa 3660
atatcataaa aatgagtgat aaatacaagg cccagatgtg gttgcataag gtttttatgc 3720
atgtttgttg tatacttcct tatgcttctt ttaaattgtg tgtgctctgc ttcaatgtag 3780
tcagaattag ctgcttctat gtttcatagt tggggtcata gatgtttcct tgccttgttg 3840
atgtggacat gagccatttg aggggagagg gaacggaaat aaaggagtta tttgtaatga 3900
aaaaaaaaaa aaaaaaaaaa aaaaaa 3926

<210> 10
<211> 4679
<212> DNA
<213> Homo sapiens

<400> 10
ggcggcgcgg cgcggcgctc gcggctgctg cctgggaggg aggccgggca ggcggctgag 60
cggcgcggct ctcaacgtga cggggaagtg gttcgggcgg ccgcggctta ctaccccagg 120
gcgaacggac ggacgacgga ggcgggagcc ggtagccgag ccgggcgacc tagagaacga 180
gcgggtcagg ctcagcgtcg gccactctgt cggtccgctg aatgaagtgc ccgcccctct 240
gagcccggag cccggcgctt tccccgcaag atggacggtt tcgccggcag tctcgatgat 300
agtatttctg ctgcaagtac ttctgatgtt caagatcgcc tgtcagctct tgagtcacga 360
gttcagcaac aagaagatga aatcactgtg ctaaaggcgg ctttggctga tgttttgagg 420
cgtcttgcaa tctctgaaga tcatgtggcc tcagtgaaaa aatcagtctc aagtaaaggc 480
caaccaagcc ctcgagcagt tattcccatg tcctgtataa ccaatggaag tggtgcaaac 540
agaaaaccaa gtcataccag tgctgtctca attgcaggaa aagaaactct ttcatctgct 600
gctaaaagtg gtacagaaaa aaagaaagaa aaaccacaag gacagagaga aaaaaaagag 660
gaatctcatt ctaatgatca aagtccacaa attcgagcat caccttctcc ccagccctct 720
tcacaacctc tccaaataca cagacaaact ccagaaagca agaatgctac tcccaccaaa 780
agcataaaac gaccatcacc agctgaaaag tcacataatt cttgggaaaa ttcagatgat 840
agccgtaata aattgtcgaa aataccttca acacccaaat taataccaaa agttaccaaa 900
actgcagaca agcataaaga tgtcatcatc aaccaagaag gagaatatat taaaatgttt 960
atgcgcggtc ggccaattac catgttcatt ccttccgatg ttgacaacta tgatgacatc 1020
agaacggaac tgcctcctga gaagctcaaa ctggagtggg catatggtta tcgaggaaag 1080
gactgtagag ctaatgttta ccttcttccg accggggaaa tagtttattt cattgcatca 1140
gtagtagtac tatttaatta tgaggagaga actcagcgac actacctggg ccatacagac 1200
tgtgtgaaat gccttgctat acatcctgac aaaattagga ttgcaactgg acagatagct 1260
ggcgtggata aagatggaag gcctctacaa ccccacgtca gagtgtggga ttctgttact 1320
ctatccacac tgcagattat tggacttggc acttttgagc gtggagtagg atgcctggat 1380
ttttcaaaag cagattcagg tgttcattta tgtgttattg atgactccaa tgagcatatg 1440
cttactgtat gggactggca gaagaaagca aaaggagcag aaataaagac aacaaatgaa 1500
gttgttttgg ctgtggagtt tcacccaaca gatgcaaata ccataattac atgcggtaaa 1560
tctcatattt tcttctggac ctggagcggc aattcactaa caagaaaaca gggaattttt 1620
gggaaatatg aaaagccaaa atttgtgcag tgtttagcat tcttggggaa tggagatgtt 1680
cttactggag actcaggtgg agtcatgctt atatggagca aaactactgt agagcccaca 1740
cctgggaaag gacctaaagg tgtatatcaa atcagcaaac aaatcaaagc tcatgatggc 1800
agtgtgttca cactttgtca gatgagaaat gggatgttat taactggagg agggaaagac 1860
agaaaaataa ttctgtggga tcatgatctg aatcctgaaa gagaaataga ggttcctgat 1920
cagtatggca caatcagagc tgtagcagaa ggaaaggcag atcaattttt agtaggcaca 1980
tcacgaaact ttattttacg aggaacattt aatgatggct tccaaataga agtacagggt 2040
catacagatg agctttgggg tcttgccaca catcccttca aagatttgct cttgacatgt 2100
gctcaggaca ggcaggtgtg cctgtggaac tcaatggaac acaggctgga atggaccagg 2160
ctggtagatg aaccaggaca ctgtgcagat tttcatccaa gtggcacagt ggtggccata 2220

ggaacgcact caggcaggtg gtttgttctg gatgcagaaa ccagagatct agtttctatc 2280
cacacagacg ggaatgaaca gctctctgtg atgcgctact caatagatgg taccttcctg 2340
gctgtaggat ctcatgacaa ctttatttac ctctatgtag tctctgaaaa tggaagaaaa 2400
tatagcagat atggaaggtg cactggacat tccagctaca tcacacacct tgactggtcc 2460
ccagacaaca agtatataat gtctaactcg ggagactatg aaatattgta cttgtaccgc 2520
cggaagcacc aggagctgca agccatgcag atggagctgc agagccctga gtacaagctg 2580
agcaagctcc gcacctcgac catcatgacc gactacaacc ccaactactg ctttgctggc 2640
aagacctcct ccatcagtga cctgaaggag gtgccgcgga aaaacatcac cctcattcgg 2700
ggtctgggcc atggagcctt tggggaggtg tatgaaggcc aggtgtccgg aatgcccaac 2760
gacccaagcc ccctgcaagt ggctgtgaag acgctgcctg aagtgtgctc tgaacaggac 2820
gaactggatt tcctcatgga agccctgatc atcagcaaat tcaaccacca gaacattgtt 2880
cgctgcattg gggtgagcct gcaatccctg ccccggttca tcctgctgga gctcatggcg 2940
gggggagacc tcaagtcctt cctccgagag acccgccctc gcccgagcca gccctcctcc 3000
ctggccatgc tggaccttct gcacgtggct cgggacattg cctgtggctg tcagtatttg 3060
gaggaaaacc acttcatcca ccgagacatt gctgccagaa actgcctctt gacctgtcca 3120
ggccctggaa gagtggccaa gattggagac ttcgggatgg cccgagacat ctacagggcg 3180
agctactata gaaagggagg ctgtgccatg ctgccagtta agtggatgcc cccagaggcc 3240
ttcatggaag gaatattcac ttctaaaaca gacacatggt cctttggagt gctgctatgg 3300
gaaatctttt ctcttggata tatgccatac cccagcaaaa gcaaccagga agttctggag 3360
tttgtcacca gtggaggccg gatggaccca cccaagaact gccctgggcc tgtataccgg 3420
ataatgactc agtgctggca acatcagcct gaagacaggc ccaactttgc catcattttg 3480
gagaggattg aatactgcac ccaggacccg gatgtaatca acaccgcttt gccgatagaa 3540
tatggtccac ttgtggaaga ggaagagaaa gtgcctgtga ggcccaagga ccctgagggg 3600
gttcctcctc tcctggtctc tcaacaggca aaacgggagg aggagcgcag cccagctgcc 3660
ccaccacctc tgcctaccac ctcctctggc aaggctgcaa agaaacccac agctgcagag 3720
gtctctgttc gagtccctag agggccggcc gtggaagggg gacacgtgaa tatggcattc 3780
tctcagtcca accctccttc ggagttgcac agggtccacg gatccagaaa caagcccacc 3840
agcttgtgga acccaacgta cggctcctgg tttacagaga aacccaccaa aaagaataat 3900
cctatagcaa agaaggagcc acacgagagg ggtaacctgg ggctggaggg aagctgtact 3960
gtcccaccta acgttgcaac tgggagactt ccgggggcct cactgctcct agagccctct 4020
tcgctgactg ccaatatgaa ggaggtacct ctgttcaggc tacgtcactt ccccttgtgg 4080
aatgtcaatt acggctacca gcaacagggc ttgcccttag aagccgctac tgcccctgga 4140
gctggtcatt acgaggatac cattctgaaa agcaagaata gcatgaacca gcctgggccc 4200
tgagctcggt cacacactca cttctcttcc ttgggatccc taagaccgtg gaggagagag 4260
aggcaatcaa tggctccttc acaaaccaga gaccaaatgt cacgttttgt tttgtgccaa 4320
cctattttga agtaccacca aaaaagctgt attttgaaaa tgctttagaa aggttttgag 4380
catgggttca tcctattctt tcgaaagaag aaaatatcat aaaaatgagt gataaataca 4440
aggcccagat gtggttgcat aaggttttta tgcatgtttg ttgtatactt ccttatgctt 4500

cttttaaatt gtgtgtgctc tgcttcaatg tagtcagaat tagctgcttc tatgtttcat 4560

agttggggtc atagatgttt ccttgccttg ttgatgtgga catgagccat ttgaggggag 4620

agggaacgga aataaaggag ttatttgtaa tgaaaaaaaa aaaaaaaaaa aaaaaaaaa 4679

<210> 11
<211> 2473
<212> DNA
<213> Homo sapiens

<400> 11
actctgtcgg tccgctgaat gaagtgcccg cccctctaag cccggagccc ggcgctttcc 60

ccgcaagatg gacggtttcg ccggcagtct cgatgatagt atttctgctg caagtacttc 120

tgatgttcaa gatcgcctgt cagctcttga gtcacgagtt cagcaacaag aagatgaaat 180

cactgtgcta aaggcggctt tggctgatgt tttgaggcgt cttgcaatct ctgaagatca 240

tgtggcctca gtgaaaaaat cagtctcaag taaaggccaa ccaagccctc gagcagttat 300

tcccatgtcc tgtataacca atggaagtgg tgcaaacaga aaaccaagtc ataccagtgc 360

tgtctcaatt gcaggaaaag aaactctttc atctgctgct aaaagtggta cagaaaaaaa 420

gaaagaaaaa ccacaaggac agagagaaaa aaaagaggaa tctcattcta atgatcaaag 480

tccacaaatt cgagcatcac cttctcccca gccctcttca caacctctcc aaatacacag 540

acaaactcca gaaagcaaga atgctactcc caccaaaagc ataaaacgac catcaccagc 600

tgaaaagtca cataattctt gggaaaattc agatgatagc cgtaataaat tgtcgaaaat 660

accttcaaca cccaaattaa taccaaaagt taccaaaact gcagacaagc ataaagatgt 720

catcatcaac caagtgtacc gccggaagca ccaggagctg caagccatgc agatggagct 780

gcagagccct gagtacaagc tgagcaagct ccgcacctcg accatcatga ccgactacaa 840

ccccaactac tgctttgctg gcaagacctc ctccatcagt gacctgaagg aggtgccgcg 900

gaaaaacatc accctcattc ggggtctggg ccatggagcc tttggggagg tgtatgaagg 960

ccaggtgtcc ggaatgccca acgacccaag ccccctgcaa gtggctgtga agacgctgcc 1020

tgaagtgtgc tctgaacagg acgaactgga tttcctcatg gaagccctga tcatcagcaa 1080

attcaaccac cagaacattg ttcgctgcat tggggtgagc ctgcaatccc tgccccggtt 1140

catcctgctg gagctcatgg cggggggaga cctcaagtcc ttcctccgag agacccgccc 1200

tcgcccgagc cagccctcct ccctggccat gctggacctt ctgcacgtgg ctcgggacat 1260

tgcctgtggc tgtcagtatt tggaggaaaa ccacttcatc caccgagaca ttgctgccag 1320

aaactgcctc ttgacctgtc caggccctgg aagagtggcc aagattggag acttcgggat 1380

ggcccgagac atctacaggg cgagctacta tagaaaggga ggctgtgcca tgctgccagt 1440

taagtggatg cccccagagg ccttcatgga aggaatattc acttctaaaa cagacacatg 1500

gtcctttgga gtgctgctat gggaaatctt ttctcttgga tatatgccat accccagcaa 1560

aagcaaccag gaagttctgg agtttgtcac cagtggaggc cggatggacc cacccaagaa 1620

ctgccctggg cctgtatacc ggataatgac tcagtgctgg caacatcagc ctgaagacag 1680

gcccaacttt gccatcattt tggagaggat tgaatactgc acccaggacc cggatgtaat 1740

caacaccgct ttgccgatag aatatggtcc acttgtggaa gaggaagaga aagtgcctgt 1800

gaggcccaag gaccctgagg gggttcctcc tctcctggtc tctcaacagg caaaacggga 1860

ggaggagcgc agcccagctg ccccaccacc tctgcctacc acctcctctg gcaaggctgc 1920

aaagaaaccc acagctgcag aggtctctgt tcgagtccct agagggccgg ccgtggaagg 1980

gggacacgtg aatatggcat tctctcagtc caaccctcct tcggagttgc acaggtccca 2040

cggatccaga aacaagccca ccagcttgtg gaacccaacg tacggctcct ggtttacaga 2100

gaaacccacc aaaaagaata atcctatagc aaagaaggag ccacacgaga ggggtaacct 2160

ggggctggag ggaagctgta ctgtcccacc taacgttgca actgggagac ttccgggggc 2220

ctcactgctc ctagagccct cttcgctgac tgccaatatg aaggaggtac ctctgttcag 2280

gctacgtcac ttcccttgtg ggaatgtcaa ttacggctac cagcaacagg gcttgccctt 2340

agaagccgct actgcccctg gagctggtca ttacgaggat accattctga aaagcaagaa 2400

tagcatgaac cagcctgggc cctgagctcg gtcgcacact cacttctctt ccttgggatc 2460

cctaagaccg tgg 2473

<210> 12
<211> 2506
<212> DNA
<213> Homo sapiens

<400> 12
actctgtcgg tccgctgaat gaagtgcccg cccctctaag cccggagccc ggcgctttcc 60

ccgcaagatg gacggtttcg ccggcagtct cgatgatagt atttctgctg caagtacttc 120

tgatgttcaa gatcgcctgt cagctcttga gtcacgagtt cagcaacaag aagatgaaat 180

cactgtgcta aaggcggctt tggctgatgt tttgaggcgt cttgcaatct ctgaagatca 240

tgtggcctca gtgaaaaaat cagtctcaag taaaggccaa ccaagccctc gagcagttat 300

tcccatgtcc tgtataacca atggaagtgg tgcaaacaga aaaccaagtc ataccagtgc 360

tgtctcaatt gcaggaaaag aaactctttc atctgctgct aaaagtggta cagaaaaaaa 420

gaaagaaaaa ccacaaggac agagagaaaa aaaagaggaa tctcattcta atgatcaaag 480

tccacaaatt cgagcatcac cttctcccca gccctcttca caacctctcc aaatacacag 540

acaaactcca gaaagcaaga atgctactcc caccaaaagc ataaaacgac catcaccagc 600

tgaaaagtca cataattctt gggaaaattc agatgatagc cgtaataaat tgtcgaaaat 660

accttcaaca cccaaattaa taccaaaagt taccaaaact gcagacaagc ataaagatgt 720

catcatcaac caagcaaaaa tgtcaactcg cgaaaaaaac agccaagtgt accgccggaa 780

gcaccaggag ctgcaagcca tgcagatgga gctgcagagc cctgagtaca agctgagcaa 840

gctccgcacc tcgaccatca tgaccgacta caaccccaac tactgctttg ctggcaagac 900

ctcctccatc agtgacctga aggaggtgcc gcggaaaaac atcaccctca ttcggggtct 960

gggccatgga gcctttgggg aggtgtatga aggccaggtg tccggaatgc ccaacgaccc 1020

aagccccctg caagtggctg tgaagacgct gcctgaagtg tgctctgaac aggacgaact 1080

ggatttcctc atggaagccc tgatcatcag caaattcaac caccagaaca ttgttcgctg 1140

cattggggtg agcctgcaat ccctgccccg gttcatcctg ctggagctca tggcgggggg 1200

agacctcaag tccttcctcc gagagacccg ccctcgcccg agccagccct cctccctggc 1260

catgctggac cttctgcacg tggctcggga cattgcctgt ggctgtcagt atttggagga 1320

aaaccacttc atccaccgag acattgctgc cagaaactgc ctcttgacct gtccaggccc 1380

tggaagagtg gccaagattg gagacttcgg gatggcccga gacatctaca gggcgagcta 1440

ctatagaaag ggaggctgtg ccatgctgcc agttaagtgg atgcccccag aggccttcat 1500

ggaaggaata ttcacttcta aaacagacac atggtccttt ggagtgctgc tatgggaaat 1560

cttttctctt ggatatatgc cataccccag caaaagcaac caggaagttc tggagtttgt 1620

caccagtgga ggccggatgg acccacccaa gaactgccct gggcctgtat accggataat 1680

gactcagtgc tggcaacatc agcctgaaga caggcccaac tttgccatca ttttggagag 1740

gattgaatac tgcacccagg acccggatgt aatcaacacc gctttgccga tagaatatgg 1800

tccacttgtg gaagaggaag agaaagtgcc tgtgaggccc aaggaccctg agggggttcc 1860

tcctctcctg gtctctcaac aggcaaaacg ggaggaggag cgcagcccag ctgccccacc 1920

acctctgcct accacctcct ctggcaaggc tgcaaagaaa cccacagctg cagaggtctc 1980

tgttcgagtc cctagagggc cggccgtgga agggggacac gtgaatatgg cattctctca 2040

gtccaaccct ccttcggagt tgcacagggt ccacggatcc agaaacaagc ccaccagctt 2100

gtggaaccca acgtacggct cctggtttac agagaaaccc accaaaaaga ataatcctat 2160

agcaaagaag gagccacacg agaggggtaa cctggggctg gagggaagct gtactgtccc 2220

acctaacgtt gcaactggga gacttccggg ggcctcactg ctcctagagc cctcttcgct 2280

gactgccaat atgaaggagg tacctctgtt caggctacgt cacttccctt gtgggaatgt 2340

caattacggc taccagcaac agggcttgcc cttagaagcc gctactgccc ctggagctgg 2400

tcattacgag gataccattc tgaaaagcaa gaatagcatg aaccagcctg ggccctgagc 2460

tcggtcgcac actcacttct cttccttggg atccctaaga ccgtgg 2506

<210> 13
<211> 3409
<212> DNA
<213> Homo sapiens

<400> 13
actctgtcgg tccgctgaat gaagtgcccg cccctctaag cccggagccc ggcgctttcc 60

ccgcaagatg gacggtttcg ccggcagtct cgatgatagt atttctgctg caagtacttc 120

tgatgttcaa gatcgcctgt cagctcttga gtcacgagtt cagcaacaag aagatgaaat 180

cactgtgcta aaggcggctt tggctgatgt tttgaggcgt cttgcaatct ctgaagatca 240

tgtggcctca gtgaaaaaat cagtctcaag taaaggccaa ccaagccctc gagcagttat 300

tcccatgtcc tgtataacca atggaagtgg tgcaaacaga aaaccaagtc ataccagtgc 360

tgtctcaatt gcaggaaaag aaactctttc atctgctgct aaaagtggta cagaaaaaaa 420

gaaagaaaaa ccacaaggac agagagaaaa aaaagaggaa tctcattcta atgatcaaag 480

tccacaaatt cgagcatcac cttctcccca gccctcttca caacctctcc aaatacacag 540

acaaactcca gaaagcaaga atgctactcc caccaaaagc ataaaacgac catcaccagc 600

tgaaaagtca cataattctt gggaaaattc agatgatagc cgtaataaat tgtcgaaaat 660

accttcaaca cccaaattaa taccaaaagt taccaaaact gcagacaagc ataaagatgt 720

catcatcaac caagaaggag aatatattaa aatgtttatg cgcggtcggc caattaccat 780

gttcattcct tccgatgttg acaactatga tgacatcaga acggaactgc ctcctgagaa 840

gctcaaactg gagtgggcat atggttatcg aggaaaggac tgtagagcta atgtttacct 900

tcttccgacc ggggaaatag tttatttcat tgcatcagta gtagtactat ttaattatga 960
ggagagaact cagcgacact acctgggcca tacagactgt gtgaaatgcc ttgctataca 1020
tcctgacaaa attaggattg caactggaca gatagctggc gtggataaag atggaaggcc 1080
tctacaaccc cacgtcagag tgtgggattc tgttactcta tccacactgc agattattgg 1140
acttggcact tttgagcgtg gagtaggatg cctggatttt tcaaaagcag attcaggtgt 1200
tcatttatgt gttattgatg actccaatga gcatatgctt actgtatggg actggcagag 1260
gaaagcaaaa ggagcagaaa taaagacaac aaatgaagtt gttttggctg tggagtttca 1320
cccaacagat gcaaatacca taattacatg cggtaaatct catattttct tctggacctg 1380
gagcggcaat tcactaacaa gaaaacaggg aatttttggg aaatatgaaa agccaaaatt 1440
tgtgcagtgt ttagcattct tggggaatgg agatgttctt actggagact caggtggagt 1500
catgcttata tggagcaaaa ctactgtaga gcccacacct gggaaaggac ctaaaggtgt 1560
atatcaaatc agcaaacaaa tcaaagctca tgatggcagt gtgttcacac tttgtcagat 1620
gagaaatggg atgttattaa ctggaggagg gaaagacaga aaaataattc tgtgggatca 1680
tgatctgaat cctgaaagag aaatagagat atgctggatg agccctgagt acaagctgag 1740
caagctccgc acctcgacca tcatgaccga ctacaacccc aactactgct ttgctggcaa 1800
gacctcctcc atcagtgacc tgaaggaggt gccgcggaaa aacatcaccc tcattcgggg 1860
tctgggccat ggagcctttg gggaggtgta tgaaggccag gtgtccggaa tgcccaacga 1920
cccaagcccc ctgcaagtgg ctgtgaagac gctgcctgaa gtgtgctctg aacaggacga 1980
actggatttc ctcatggaag ccctgatcat cagcaaattc aaccaccaga acattgttcg 2040
ctgcattggg gtgagcctgc aatccctgcc ccggttcatc ctgctggagc tcatggcggg 2100
gggagacctc aagtccttcc tccgagagac ccgccctcgc ccgagccagc cctcctccct 2160
ggccatgctg gaccttctgc acgtggctcg ggacattgcc tgtggctgtc agtatttgga 2220
ggaaaaccac ttcatccacc gagacattgc tgccagaaac tgcctcttga cctgtccagg 2280
ccctggaaga gtggccaaga ttggagactt cgggatggcc cgagacatct acagggcgag 2340
ctactataga aagggaggct gtgccatgct gccagttaag tggatgcccc cagaggcctt 2400
catggaagga atattcactt ctaaaacaga cacatggtcc tttggagtgc tgctatggga 2460
aatcttttct cttggatata tgccataccc cagcaaaagc aaccaagaag ttctggagtt 2520
tgtcaccagt ggaggccgga tggacccacc caagaactgc cctgggcctg tataccggat 2580
aatgactcag tgctggcaac atcagcctga agacaggccc aactttgcca tcattttgga 2640
gaggattgaa tactgcaccc aggacccgga tgtaatcaac accgctttgc cgatagaata 2700
tggtccactt gtggaagagg aagagaaagt gcctgtgagg cccaaggacc ctgagggggt 2760
tcctcctctc ctggtctctc aacaggcaaa acgggaggag gagcgcagcc cagctgcccc 2820
accacctctg cctaccacct cctctggcaa ggctgcaaag aaacccacag ctgcagaggt 2880
ctctgttcga gtccctagag ggccggccgt ggaaggggga cacgtgaata tggcattctc 2940
tcagtccaac cctccttcgg agttgcacag ggtccacgga tccagaaaca agcccaccag 3000
cttgtggaac ccaacgtacg gctcctggtt tacagagaaa cccaccaaaa agaataatcc 3060
tatagcaaag aaggagccac acgagagggg taacctgggg ctggagggaa gctgtactgt 3120

cccacctaac gttgcaactg ggagacttcc gggggcctca ctgctcctag agccctcttc 3180

gctgactgcc aatatgaagg aggtacctct gttcaggcta cgtcacttcc cttgtgggaa 3240

tgtcaattac ggctaccagc aacagggctt gcccttagaa gccgctactg cccctggagc 3300

tggtcattac gaggatacca ttctgaaaag caagaatagc atgaaccagc ctgggccctg 3360

agctcggtcg cacactcact tctcttcctt gggatcccta agaccgtgg 3409

<210> 14
<211> 2014
<212> DNA
<213> Homo sapiens

<400> 14
actctgtcgg tccgctgaat gaagtgcccg cccctctaag cccggagccc ggcgctttcc 60

ccgcaagatg gacggtttcg ccggcagtct cgatgatagt atttctgctg caagtacttc 120

tgatgttcaa gatcgcctgt cagctcttga gtcacgagtt cagcaacaag aagatgaaat 180

cactgtgcta aaggcggctt tggctgatgt tttgaggcgt cttgcaatct ctgaagatca 240

tgtggcctca gtgaaaaaat cagtctcaag taaagtgtac cgccggaagc accaggagct 300

gcaagccatg cagatggagc tgcagagccc tgagtacaag ctgagcaagc tccgcacctc 360

gaccatcatg accgactaca accccaacta ctgctttgct ggcaagacct cctccatcag 420

tgacctgaag gaggtgccgc ggaaaaacat caccctcatt cggggtctgg gccatggagc 480

ctttggggag gtgtatgaag gccaggtgtc cggaatgccc aacgacccaa gccccctgca 540

agtggctgtg aagacgctgc ctgaagtgtg ctctgaacag gacgaactgg atttcctcat 600

ggaagccctg atcatcagca aattcaacca ccagaacatt gttcgctgca ttggggtgag 660

cctgcaatcc ctgccccggt tcatcctgct ggagctcatg gcggggggag acctcaagtc 720

cttcctccga gagacccgcc ctcgcccgag ccagccctcc tccctggcca tgctggacct 780

tctgcacgtg gctcgggaca ttgcctgtgg ctgtcagtat ttggaggaaa accacttcat 840

ccaccgagac attgctgcca gaaactgcct cttgacctgt ccaggccctg gaagagtggc 900

caagattgga gacttcggga tggcccgaga catctacagg gcgagctact atagaaaggg 960

aggctgtgcc atgctgccag ttaagtggat gcccccagag gccttcatgg aaggaatatt 1020

cacttctaaa acagacacat ggtcctttgg agtgctgcta tgggaaatct tttctcttgg 1080

atatatgcca taccccagca aaagcaacca ggaagttctg gagtttgtca ccagtggagg 1140

ccggatggac ccacccaaga actgccctgg gcctgtatac cggataatga ctcagtgctg 1200

gcaacatcag cctgaagaca ggcccaactt tgccatcatt ttggagagga ttgaatactg 1260

cacccaggac ccggatgtaa tcaacaccgc tttgccgata gaatatggtc cacttgtgga 1320

agaggaagag aaagtgcctg tgaggcccaa ggaccctgag ggggttcctc ctctcctggt 1380

ctctcaacag gcaaaacggg aggaggagcg cagcccagct gccccaccac ctctgcctac 1440

cacctcctct ggcaaggctg caaagaaacc cacagctgca gaggtctctg ttcgagtccc 1500

tagagggccg gccgtggaag ggggacacgt gaatatggca ttctctcagt ccaaccctcc 1560

ttcggagttg cacagggtcc acggatccag aaacaagccc accagcttgt ggaacccaac 1620

gtacggctcc tggtttacag agaaacccac caaaaagaat aatcctatag caaagaagga 1680

gccacacgag aggggtaacc tggggctgga gggaagctgt actgtcccac ctaacgttgc 1740

aactgggaga cttccggggg cctcactgct cctagagccc tcttcgctga ctgccaatat 1800

gaaggaggta cctctgttca ggctacgtca cttcccttgt gggaatgtca attacggcta 1860

ccagcaacag ggcttgccct tagaagccgc tactgcccct ggagctggtc attacgagga 1920

taccattctg aaaagcaaga atagcatgaa ccagcctggg ccctgagctc ggtcgcacac 1980

tcacttctct tccttgggat ccctaagacc gtgg 2014

<210> 15
<211> 2131
<212> DNA
<213> Homo sapiens

<400> 15
actctgtcgg tccgctgaat gaagtgcccg cccctctaag cccggagccc ggcgctttcc 60

ccgcaagatg gacggtttcg ccggcagtct cgatgatagt atttctgctg caagtacttc 120

tgatgttcaa gatcgcctgt cagctcttga gtcacgagtt cagcaacaag aagatgaaat 180

cactgtgcta aaggcggctt tggctgatgt tttgaggcgt cttgcaatct ctgaagatca 240

tgtggcctca gtgaaaaaat cagtctcaag taaaggttca gagctcaggg gaggatatgg 300

agatccaggg aggcttcctg taggaagtgg cctgtgtagt gcttcaaggg ccaggctgcc 360

aggccatgtt gcagctgacc acccacctgc agtgtaccgc cggaagcacc aggagctgca 420

agccatgcag atggagctgc agagccctga gtacaagctg agcaagctcc gcacctcgac 480

catcatgacc gactacaacc ccaactactg ctttgctggc aagacctcct ccatcagtga 540

cctgaaggag gtgccgcgga aaaacatcac cctcattcgg ggtctgggcc atggagcctt 600

tggggaggtg tatgaaggcc aggtgtccgg aatgcccaac gacccaagcc ccctgcaagt 660

ggctgtgaag acgctgcctg aagtgtgctc tgaacaggac gaactggatt tcctcatgga 720

agccctgatc atcagcaaat tcaaccacca gaacattgtt cgctgcattg gggtgagcct 780

gcaatccctg ccccggttca tcctgctgga gctcatggcg gggggagacc tcaagtcctt 840

cctccgagag acccgccctc gcccgagcca gccctcctcc ctggccatgc tggaccttct 900

gcacgtggct cgggacattg cctgtggctg tcagtatttg gaggaaaacc acttcatcca 960

ccgagacatt gctgccagaa actgcctctt gacctgtcca ggccctggaa gagtggccaa 1020

gattggagac ttcgggatgg cccgagacat ctacagggcg agctactata gaaagggagg 1080

ctgtgccatg ctgccagtta agtggatgcc cccagaggcc ttcatggaag gaatattcac 1140

ttctaaaaca gacacatggt cctttggagt gctgctatgg gaaatctttt ctcttggata 1200

tatgccatac cccagcaaaa gcaaccagga agttctggag tttgtcacca gtggaggccg 1260

gatggaccca cccaagaact gccctgggcc tgtataccgg ataatgactc agtgctggca 1320

acatcagcct gaagacaggc ccaactttgc catcattttg gagaggattg aatactgcac 1380

ccaggacccg gatgtaatca acaccgcttt gccgatagaa tatggtccac ttgtggaaga 1440

ggaagagaaa gtgcctgtga ggcccaagga ccctgagggg gttcctcctc tcctggtctc 1500

tcaacaggca aaacgggagg aggagcgcag cccagctgcc ccaccacctc tgcctaccac 1560

ctcctctggc aaggctgcaa agaaacccac agctgcagag gtctctgttc gagtccctag 1620

agggccggcc gtggaagggg gacacgtgaa tatggcattc tctcagtcca accctccttc 1680

ggagttgcac agggtccacg gatccagaaa caagcccacc agcttgtgga acccaacgta 1740

cggctcctgg tttacagaga aacccaccaa aaagaataat cctatagcaa agaaggagcc 1800

acacgagagg ggtaacctgg ggctggaggg aagctgtact gtcccaccta acgttgcaac 1860

tgggagactt ccgggggcct cactgctcct agagccctct tcgctgactg ccaatatgaa 1920

ggaggtacct ctgttcaggc tacgtcactt cccttgtggg aatgtcaatt acggctacca 1980

gcaacagggc ttgcccttag aagccgctac tgcccctgga gctggtcatt acgaggatac 2040

cattctgaaa agcaagaata gcatgaacca gcctgggccc tgagctcggt cgcacactca 2100

cttctcttcc ttgggatccc taagaccgtg g 2131

<210> 16
<211> 3365
<212> DNA
<213> Homo sapiens

<400> 16
tactctgtcg gtccgctgaa tgaagtgccc gcccctctaa gcccggagcc cggcgctttc 60

cccgcaagat ggacggtttc gccggcagtc tcgatgatag tatttctgct gcaagtactt 120

ctgatgttca agatcgcctg tcagctcttg agtcacgagt tcagcaacaa gaagatgaaa 180

tcactgtgct aaaggcggct ttggctgatg ttttgaggcg tcttgcaatc tctgaagatc 240

atgtggcctc agtgaaaaaa tcagtctcaa gtaaaggcca accaagccct cgagcagtta 300

ttcccatgtc ctgtataacc aatggaagtg gtgcaaacag aaaaccaagt cataccagtg 360

ctgtctcaat tgcaggaaaa gaaactcttt catctgctgc taaaagtggt acagaaaaaa 420

agaaagaaaa accacaagga cagagagaaa aaaaagagga atctcattct aatgatcaaa 480

gtccacaaat tcgagcatca ccttctcccc agccctcttc acaacctctc caaatacaca 540

gacaaactcc agaaagcaag aatgctactc ccaccaaaag cataaaacga ccatcaccag 600

ctgaaaagtc acataattct tgggaaaatt cagatgatag ccgtaataaa ttgtcgaaaa 660

taccttcaac acccaaatta ataccaaaag ttaccaaaac tgcagacaag cataaagatg 720

tcatcatcaa ccaagaagga gaatatatta aaatgtttat gcgcggtcgg ccaattacca 780

tgttcattcc ttccgatgtt gacaactatg atgacatcag aacggaactg cctcctgaga 840

agctcaaact ggagtgggca tatggttatc gaggaaagga ctgtagagct aatgtttacc 900

ttcttccgac cggggaaata gtttatttca ttgcatcagt agtagtacta tttaattatg 960

aggagagaac tcagcgacac tacctgggcc atacagactg tgtgaaatgc cttgctatac 1020

atcctgacaa aattaggatt gcaactggac agatagctgg cgtggataaa gatggaaggc 1080

ctctacaacc ccacgtcaga gtgtgggatt ctgttactct atccacactg cagattattg 1140

gacttggcac ttttgagcgt ggagtaggat gcctggattt ttcaaaagca gattcaggtg 1200

ttcatttatg tgttattgat gactccaatg agcatatgct tactgtatgg gactggcaga 1260

ggaaagcaaa aggagcagaa ataaagacaa caaatgaagt tgttttggct gtggagtttc 1320

acccaacaga tgcaaatacc ataattacat gcggtaaatc tcatattttc ttctggacct 1380

ggagcggcaa ttcactaaca agaaaacagg gaatttttgg gaaatatgaa aagccaaaat 1440

ttgtgcagtg tttagcattc ttggggaatg gagatgttct tactggagac tcaggtggag 1500

tcatgcttat atggagcaaa actactgtag agcccacacc tgggaaagga cctaaaggaa 1560

gtggcctgtg tagtgcttca agggccaggc tgccaggcca tgttgcagct gaccacccac 1620

ctgcagtgta ccgccggaag caccaggagc tgcaagccat gcagatggag ctgcagagcc 1680
ctgagtacaa gctgagcaag ctccgcacct cgaccatcat gaccgactac aaccccaact 1740
actgctttgc tggcaagacc tcctccatca gtgacctgaa ggaggtgccg cggaaaaaca 1800
tcaccctcat tcggggtctg ggccatggag cctttgggga ggtgtatgaa ggccaggtgt 1860
ccggaatgcc caacgaccca agccccctgc aagtggctgt gaagacgctg cctgaagtgt 1920
gctctgaaca ggacgaactg gatttcctca tggaagccct gatcatcagc aaattcaacc 1980
accagaacat tgttcgctgc attggggtga gcctgcaatc cctgccccgg ttcatcctgc 2040
tggagctcat ggcgggggga gacctcaagt ccttcctccg agagacccgc cctcgcccga 2100
gccagccctc ctccctggcc atgctggacc ttctgcacgt ggctcgggac attgcctgtg 2160
gctgtcagta tttggaggaa aaccacttca tccaccgaga cattgctgcc agaaactgcc 2220
tcttgacctg tccaggccct ggaagagtgg ccaagattgg agacttcggg atggcccgag 2280
acatctacag ggcgagctac tatagaaagg gaggctgtgc catgctgcca gttaagtgga 2340
tgcccccaga ggccttcatg gaaggaatat tcacttctaa aacagacaca tggtcctttg 2400
gagtgctgct atgggaaatc ttttctcttg gatatatgcc ataccccagc aaaagcaacc 2460
aggaagttct ggagtttgtc accagtggag gccggatgga cccacccaag aactgccctg 2520
ggcctgtata ccggataatg actcagtgct ggcaacatca gcctgaagac aggcccaact 2580
ttgccatcat tttggagagg attgaatact gcacccagga cccggatgta atcaacaccg 2640
ctttgccgat agaatatggt ccacttgtgg aagaggaaga gaaagtgcct gtgaggccca 2700
aggaccctga gggggttcct cctctcctgg tctctcaaca ggcaaaacgg gaggaggagc 2760
gcagcccagc tgccccacca cctctgccta ccacctcctc tggcaaggct gcaaagaaac 2820
ccacagctgc agaggtctct gttcgagtcc ctagagggcc ggccgtggaa gggggacacg 2880
tgaatatggc attctctcag tccaaccctc cttcggagtt gcacagggtc cacggatcca 2940
gaaacaagcc caccagcttg tggaacccaa cgtacggctc ctggtttaca gagaaaccca 3000
ccaaaaagaa taatcctata gcaaagaagg agccacacga gaggggtaac ctggggctgg 3060
agggaagctg tactgtccca cctaacgttg caactgggag acttccgggg gcctcactgc 3120
tcctagagcc ctcttcgctg actgccaata tgaaggaggt acctctgttc aggctacgtc 3180
acttcccttg tgggaatgtc aattacggct accagcaaca gggcttgccc ttagaagccg 3240
ctactgcccc tggagctggt cattacgagg ataccattct gaaaagcaag aatagcatga 3300
accagcctgg gccctgagct cggtcgcaca ctcacttctc ttccttggga tccctaagac 3360
cgtgg 3365

<210> 17
<211> 3435
<212> DNA
<213> Homo sapiens

<400> 17
tactctgtcg gtccgctgaa tgaagtgccc gcccctctaa gcccggagcc cggcgctttc 60
cccgcaagat ggacggtttc gccggcagtc tcgatgatag tatttctgct gcaagtactt 120
ctgatgttca agatcgcctg tcagctcttg agtcacgagt tcagcaacaa gaagatgaaa 180
tcactgtgct aaaggcggct ttggctgatg ttttgaggcg tcttgcaatc tctgaagatc 240

atgtggcctc agtgaaaaaa tcagtctcaa gtaaaggcca accaagccct cgagcagtta 300
ttcccatgtc ctgtataacc aatggaagtg gtgcaaacag aaaaccaagt cataccagtg 360
ctgtctcaat tgcaggaaaa gaaactcttt catctgctgc taaaagtggt acagaaaaaa 420
agaaagaaaa accacaagga cagagagaaa aaaaagagga atctcattct aatgatcaaa 480
gtccacaaat tcgagcatca ccttctcccc agccctcttc acaacctctc caaatacaca 540
gacaaactcc agaaagcaag aatgctactc ccaccaaaag cataaaacga ccatcaccag 600
ctgaaaagtc acataattct tgggaaaatt cagatgatag ccgtaataaa ttgtcgaaaa 660
taccttcaac acccaaatta ataccaaaag ttaccaaaac tgcagacaag cataaagatg 720
tcatcatcaa ccaagaagga gaatatatta aaatgtttat gcgcggtcgg ccaattacca 780
tgttcattcc ttccgatgtt gacaactatg atgacatcag aacggaactg cctcctgaga 840
agctcaaact ggagtgggca tatggttatc gaggaaagga ctgtagagct aatgtttacc 900
ttcttccgac cggggaaata gtttatttca ttgcatcagt agtagtacta tttaattatg 960
aggagagaac tcagcgacac tacctgggcc atacagactg tgtgaaatgc cttgctatac 1020
atcctgacaa aattaggatt gcaactggac agatagctgg cgtggataaa gatggaaggc 1080
ctctacaacc ccacgtcaga gtgtgggatt ctgttactct atccacactg cagattattg 1140
gacttggcac ttttgagcgt ggagtaggat gcctggattt ttcaaaagca gattcaggtg 1200
ttcatttatg tgttattgat gactccaatg agcatatgct tactgtatgg gactggcaga 1260
ggaaagcaaa aggagcagaa ataaagacaa caaatgaagt tgttttggct gtggagtttc 1320
acccaacaga tgcaaatacc ataattacat gcggtaaatc tcatattttc ttctggacct 1380
ggagcggcaa ttcactaaca agaaaacagg gaatttttgg gaaatatgaa aagccaaaat 1440
ttgtgcagtg tttagcattc ttggggaatg gagatgttct tactggagac tcaggtggag 1500
tcatgcttat atggagcaaa actactgtag agcccacacc tgggaaagga cctaaaggtg 1560
tatatcaaat cagcaaacaa atcaaagctc atgatggcag tgtgttcaca ctttgtcaga 1620
tgagaaatgg gatgttatta actggaggag ggaaagacag aaaaataatt ctgtgggatc 1680
atgatctgaa tcctgaaaga gaaatagagc accaggagct gcaagccatg cagatggagc 1740
tgcagagccc tgagtacaag ctgagcaagc tccgcacctc gaccatcatg accgactaca 1800
accccaacta ctgctttgct ggcaagacct cctccatcag tgacctgaag gaggtgccgc 1860
ggaaaaacat caccctcatt cggggtctgg gccatggagc ctttggggag gtgtatgaag 1920
gccaggtgtc cggaatgccc aacgacccaa gccccctgca agtggctgtg aagacgctgc 1980
ctgaagtgtg ctctgaacag gacgaactgg atttcctcat ggaagccctg atcatcagca 2040
aattcaacca ccagaacatt gttcgctgca ttggggtgag cctgcaatcc ctgccccggt 2100
tcatcctgct ggagctcatg gcggggggag acctcaagtc cttcctccga gagacccgcc 2160
ctcgcccgag ccagccctcc tccctggcca tgctggacct tctgcacgtg gctcgggaca 2220
ttgcctgtgg ctgtcagtat ttggaggaaa accacttcat ccaccgagac attgctgcca 2280
gaaactgcct cttgacctgt ccaggccctg gaagagtggc caagattgga gacttcggga 2340
tggcccgaga catctacagg gcgagctact atagaaaggg aggctgtgcc atgctgccag 2400
ttaagtggat gcccccagag gccttcatgg aaggaatatt cacttctaaa acagacacat 2460
ggtcctttgg agtgctgcta tgggaaatct tttctcttgg atatatgcca taccccagca 2520

aaagcaacca ggaagttctg gagtttgtca ccagtggagg ccggatggac ccacccaaga 2580

actgccctgg gcctgtatac cggataatga ctcagtgctg gcaacatcag cctgaagaca 2640

ggcccaactt tgccatcatt ttggagagga ttgaatactg cacccaggac ccggatgtaa 2700

tcaacaccgc tttgccgata gaatatggtc cacttgtgga agaggaagag aaagtgcctg 2760

tgaggcccaa ggaccctgag ggggttcctc ctctcctggt ctctcaacag gcaaaacggg 2820

aggaggagcg cagcccagct gccccaccac ctctgcctac cacctcctct ggcaaggctg 2880

caaagaaacc cacagctgca gaggtctctg ttcgagtccc tagagggccg gccgtggaag 2940

ggggacacgt gaatatggca ttctctcagt ccaaccctcc ttcggagttg cacaaggtcc 3000

acggatccag aaacaagccc accagcttgt ggaacccaac gtacggctcc tggtttacag 3060

agaaacccac caaaaagaat aatcctatag caaagaagga gccacacgac aggggtaacc 3120

tggggctgga gggaagctgt actgtcccac ctaacgttgc aactgggaga cttccggggg 3180

cctcactgct cctagagccc tcttcgctga ctgccaatat gaaggaggta cctctgttca 3240

ggctacgtca cttcccttgt gggaatgtca attacggcta ccagcaacag ggcttgccct 3300

tagaagccgc tactgcccct ggagctggtc attacgagga taccattctg aaaagcaaga 3360

atagcatgaa ccagcctggg ccctgagctc ggtcgcacac tcacttctct tccttgggat 3420

ccctaagacc gtgga 3435

<210> 18
<211> 4479
<212> DNA
<213> Homo sapiens

<400> 18
tgcgagaaag atggcggacc tggccgagtg caacatcaaa gtgatgtgtc gcttcagacc 60

tctcaacgag tctgaagtga accgcggcga caagtacatc gccaagtttc agggagaaga 120

cacggtcgtg atcgcgtcca agccttatgc atttgatcgg gtgttccagt caagcacatc 180

tcaagagcaa gtgtataatg actgtgcaaa gaagattgtt aaagatgtac ttgaaggata 240

taatggaaca atatttgcat atggacaaac atcctctggg aagacacaca caatggaggg 300

taaacttcat gatccagaag gcatgggaat tattccaaga atagtgcaag atatttttaa 360

ttatatttac tccatggatg aaaatttgga atttcatatt aaggtttcat attttgaaat 420

atatttggat aagataaggg acctgttaga tgtttcaaag accaaccttt cagttcatga 480

agacaaaaac cgagttccct atgtaaaggg gtgcacagag cgttttgtat gtagtccaga 540

tgaagttatg gataccatag atgaaggaaa atccaacaga catgtagcag ttacaaatat 600

gaatgaacat agctctagga gtcacagtat atttcttatt aatgtcaaac aagagaacac 660

acaaacggaa caaaagctga gtggaaaact ttatctggtt gatttagctg gtagtgaaaa 720

ggttagtaaa actggagctg aaggtgctgt gctggatgaa gctaaaaaca tcaacaagtc 780

actttctgct cttggaaatg ttatttctgc tttggctgag ggtagtacat atgttccata 840

tcgagatagt aaaatgacaa gaatccttca agattcatta ggtggcaact gtagaaccac 900

tattgtaatt tgctgctctc catcatcata caatgagtct gaaacaaaat ctacactctt 960

atttggccaa agggccaaaa caattaagaa cacagtttgt gtcaatgtgg agttaactgc 1020

agaacagtgg aaaaagaagt atgaaaaaga aaaagaaaaa aataagatcc tgcggaacac 1080

tattcagtgg cttgaaaatg agctcaacag atggcgtaat ggggagacgg tgcctattga 1140
tgaacagttt gacaaagaga aagccaactt ggaagctttc acagtggata aagatattac 1200
tcttaccaat gataaaccag caaccgcaat tggagttata ggaaatttta ctgatgctga 1260
aagaagaaag tgtgaagaag aaattgctaa attatacaaa cagcttgatg acaaggatga 1320
agaaattaac cagcaaagtc aactggtaga gaaactgaag acgcaaatgt tggatcagga 1380
ggagcttttg gcatctacca gaagggatca agacaatatg caagctgagc tgaatcgcct 1440
tcaagcagaa aatgatgcct ctaaagaaga agtgaaagaa gttttacagg ccctagaaga 1500
acttgctgtc aattatgatc agaagtctca ggaagttgaa gacaaaacta aggaatatga 1560
attgcttagt gatgaattga atcagaaatc ggcaacttta gcgagtatag atgctgagct 1620
tcagaaactt aaggaaatga ccaaccacca gaaaaaacga gcagctgaga tgatggcatc 1680
tttactaaaa gaccttgcag aaataggaat tgctgtggga aataatgatg taaagcagcc 1740
tgagggaact ggcatgatag atgaagagtt cactgttgca agactctaca ttagcaaaat 1800
gaagtcagaa gtaaaaacca tggtgaaacg ttgcaagcag ttagaaagca cacaaactga 1860
gagcaacaaa aaaatggaag aaaatgaaaa ggagttagca gcatgtcagc ttcgtatctc 1920
tcaacatgaa gccaaaatca agtcattgac tgaatacctt caaaatgtgg aacaaaagaa 1980
aagacagttg gaggaatctg tcgatgccct cagtgaagaa ctagtccagc ttcgagcaca 2040
agagaaagtc catgaaatgg aaaaggagca cttaaataag gttcagactg caaatgaagt 2100
taagcaagct gttgaacagc agatccagag ccatagagaa actcatcaaa aacagatcag 2160
tagtttgaga gatgaagtag aagcaaaagc aaaacttatt actgatcttc aagaccaaaa 2220
ccagaaaatg atgttagagc aggaacgtct aagagtagaa catgagaagt tgaaagccac 2280
agatcaggaa aagagcagaa aactacatga acttacggtt atgcaagata gacgagaaca 2340
agcaagacaa gacttgaagg gtttggaaga gacagtggca aaagaacttc agactttaca 2400
caacctgcgc aaactctttg ttcaggacct ggctacaaga gttaaaaaga gtgctgagat 2460
tgattctgat gacaccggag gcagcgctgc tcagaagcaa aaaatctcct ttcttgaaaa 2520
taatcttgaa cagctcacta aagtgcacaa acagttggta cgtgataatg cagatctccg 2580
ctgtgaactt cctaagttgg aaaagcgact tcgagctaca gctgagagag tgaaagcttt 2640
ggaatcagca ctgaaagaag ctaaagaaaa tgcatctcgt gatcgcaaac gctatcagca 2700
agaagtagat cgcataaagg aagcagtcag gtcaaagaat atggccagaa gagggcattc 2760
tgcacagatt gtgtaccgcc ggaagcacca ggagctgcaa gccatgcaga tggagctgca 2820
gagccctgag tacaagctga gcaagctccg cacctcgacc atcatgaccg actacaaccc 2880
caactactgc tttgctggca agacctcctc catcagtgac ctgaaggagg tgccgcggaa 2940
aaacatcacc ctcattcggg gtctgggcca tggcgccttt ggggaggtgt atgaaggcca 3000
ggtgtccgga atgcccaacg acccaagccc cctgcaagtg gctgtgaaga cgctgcctga 3060
agtgtgctct gaacaggacg aactggattt cctcatggaa gccctgatca tcagcaaatt 3120
caaccaccag aacattgttc gctgcattgg ggtgagcctg caatccctgc cccggttcat 3180
cctgctggag ctcatggcgg ggggagacct caagtccttc ctccgagaga cccgccctcg 3240
cccgagccag ccctcctccc tggccatgct ggaccttctg cacgtggctc gggacattgc 3300

ctgtggctgt cagtatttgg aggaaaacca cttcatccac cgagacattg ctgccagaaa 3360
ctgcctcttg acctgtccag gccctggaag agtggccaag attggagact tcgggatggc 3420
ccgagacatc tacagggcga gctactatag aaagggaggc tgtgccatgc tgccagttaa 3480
gtggatgccc ccagaggcct tcatggaagg aatattcact tctaaaacag acacatggtc 3540
ctttggagtg ctgctatggg aaatcttttc tcttggatat atgccatacc ccagcaaaag 3600
caaccaggaa gttctggagt ttgtcaccag tggaggccgg atggacccac ccaagaactg 3660
ccctgggcct gtataccgga taatgactca gtgctggcaa catcagcctg aagacaggcc 3720
caactttgcc atcattttgg agaggattga atactgcacc caggacccgg atgtaatcaa 3780
caccgctttg ccgatagaat atggtccact tgtggaagag gaagagaaag tgcctgtgag 3840
gcccaaggac cctgaggggg ttcctcctct cctggtctct caacaggcaa aacggaggga 3900
ggagcgcagc ccagctgccc caccacctct gcctaccacc tcctctggca aggctgcaaa 3960
gaaacccaca gctgcagagg tctctgttcg agtccctaga gggccggccg tggaaggggg 4020
acacgtgaat atggcattct ctcagtccaa ccctccttcg gagttgcaca aggtccacgg 4080
atccagaaac aagcccacca gcttgtggaa cccaacgtac ggctcctggt ttacagagaa 4140
acccaccaaa aagaataatc ctatagcaaa gaaggagcca cacgacaggg gtaacctggg 4200
gctggaggga agctgtactg tcccacctaa cgttgcaact gggagacttc cgggggcctc 4260
actgctccta gagccctctt cgctgactgc caatatgaag gaggtacctc tgttcaggct 4320
acgtcacttc ccttgtggga atgtcaatta cggctaccag caacagggct tgcccttaga 4380
agccgctact gcccctggag ctggtcatta cgaggatacc attctgaaaa gcaagaatag 4440
catgaaccag cctgggccct gagctcggtc gcacactca 4479

<210> 19
<211> 24
<212> DNA
<213> Artificial Sequence

<220>
<223> ALK 5' specific probe

<400> 19
tctccatgtg agctccgaat gtcc 24

<210> 20
<211> 21
<212> DNA
<213> Artificial Sequence

<220>
<223> primer for detection of ALK 3' region

<400> 20
atgctgccag ttaagtggat g 21

<210> 21
<211> 21
<212> DNA
<213> Artificial Sequence

<220>
<223> primer for detection of ALK 3' region

<400> 21
actggtgaca aactccagaa c 21

<210>  22
<211>  25
<212>  DNA
<213>  Artificial Sequence

<220>
<223>  ALK 3' specific probe

<400>  22
tatgccatac cccagcaaaa gcaac                                                25


<210>  23
<211>  20
<212>  DNA
<213>  Artificial Sequence

<220>
<223>  primer for measurement of housekeeping gene TBP

<400>  23
cttggcgtgt gaagataacc                                                      20


<210>  24
<211>  20
<212>  DNA
<213>  Artificial Sequence

<220>
<223>  primer for measurement of housekeeping gene TBP

<400>  24
tgctgccttt gttgctcttc                                                      20


<210>  25
<211>  24
<212>  DNA
<213>  Artificial Sequence

<220>
<223>  TBP gene specific probe

<400>  25
ccttacgctc agggcttggc ctcc                                                 24

## Description

### TECHNICAL FIELD

The present invention relates to a method for measuring a fusion gene(s) which may be present in a living body.

### BACKGROUND ART

It is known that fusion genes may cause cancer in some cancer patients. For example, anaplastic lymphoma kinase *(ALK)* gene, one of the receptor tyrosine kinases, is known to become oncogenic by fusion with various genes (Non-patent Document 5).

*EML4-ALK* fusion gene is a gene generated by fusion between a portion of 5'-region of Echinoderm Microtubule-associated protein-Like 4 *(EML4)* gene and 3'-region of *ALK* gene, and is known to cause some non-small-cell lung cancers (Patent Document 1, Non-patent Document 1). As a method for detection of *EML4-ALK* fusion gene, a method in which *EML4-ALK* fusion gene mRNA is amplified by RT-PCR has been reported (Non-patent Document 2).

It is known that *EML4-ALK* fusion gene includes at least 9 variants whose fusion points are different from one another (Non-patent Document 3). Therefore, if it is desired to detect the expression of all of the fusion genes efficiently and simply by using the known method, complex multiplexing is required for modifying the known method into a system in which many primers and probes are used. A lot of costs and time are needed for such multiplexing, which is problematic. In addition, besides *EML4* gene, a plurality of genes have been reported as a fusion partner of *ALK* gene (Non-patent Document 4, Non-patent Document 5), and therefore it is highly likely that new fusion genes will be found. It is impossible to detect unknown fusion genes whose sequences are not yet identified by the known method in which specific primers and/or probes are designed for individual fusion genes.

### PRIOR ART DOCUMENTS

### PATENT DOCUMENTS

Patent Document 1: JP 2008-295444 A

### NON-PATENT DOCUMENTS

Non-patent Document 1: Nature 2007; 448:561-566
Non-patent Document 2: Clin. Can. Res. 2008; 14:6618-6624
Non-patent Document 3: J. Clin. Oncol. 2009; 27:1-4
Non-patent Document 4: Clin. Can. Res. 2009; 15:3143-3149
Non-patent Document 5: Biochem. J. 2009; 420:345-361

### SUMMARY OF THE INVENTION

### PROBLEMS TO BE SOLVED BY THE INVENTION

Accordingly, an object of the present invention is to provide a simple detection system for fusion genes including those not yet known.

### MEANS FOR SOLVING THE PROBLEMS

The present inventor has intensively studied to find that the nature of the *EML4-ALK* fusion gene is "X (an arbitrary gene) + 3'-region (comprising kinase domain) of *ALK* gene", and that the presence of any fusion genes can be easily and rapidly detected regardless of the kind of their fusion partners by measuring expression level of regions upstream and downstream of the fusion point *in ALK* gene. Furthermore, he has examined more than 50 kinds of primer sets to find combinations of the primers which can attain simultaneous amplification including internal control while reducing non-specific amplification, thereby completing the present invention.

That is, the present invention provides a method for measuring a fusion gene(s), which is applied to a sample separated from a living body, said method comprising: measuring expressions of a 5'-region and a 3'-region of one of the component genes of the fusion gene(s) which may be present in the living body, wherein said 5'-region is upstream of and said 3'-region is downstream of a fusion point in said one of the component genes; and comparing the expression of the 5'-region with the expression of the 3'-region. The present invention also provides a reagent for detection of a fusion gene(s) formed by fusion with *ALK* gene, said reagent comprising a 5'-region detection primer set composed of the base sequences shown in SEQ ID NOs: 3 and 4 of the SEQUENCE LISTING, respectively, and a 3'-region detection primer set composed of the base sequences shown in SEQ ID NOs: 5 and 6, respectively. The present invention further provides a reagent for detection of a fusion gene(s) formed by fusion *with ALK* gene, said reagent comprising a 5'-region detection primer set composed of the base sequences shown in SEQ ID NOs: 3 and 4 of the SEQUENCE LISTING, respectively, and a 3'-region detection primer set composed of the base sequences shown in SEQ ID NOs: 20 and 21, respectively.

### EFFECTS OF THE INVENTION

By the present invention, a novel method which can comprehensively detect fusion genes which may be present in living bodies regardless of the kind of the fusion partner was provided. According to the present invention, the expression levels of just two regions of a gene are measured, so that any fusion genes generated by fusion with any fusion partner can be detected, thereby greatly reducing time and costs spent on detection of various kinds of fusion genes. For example, as an *ALK* fusion gene, those generated by fusion between an *ALK* 3'-region comprising the kinase domain of *ALK* gene and a fusion partner such as *EML4* gene or the like are known. The method of the present invention can detect any fusion genes in which an upstream region of *ALK* gene is lost, and therefore can detect even novel fusion genes which have not been specifically identified yet, which is one of the great advantages of the present invention. Particularly, by using the specific primer sets the present inventor has completed, PCR can be carried out in a single tube, including an internal control. Therefore, the present invention is very advantageous as a simple and rapid detection system for *ALK* fusion genes. The presence of fusion genes such as *EML4-ALK* fusion gene and the like has been confirmed in some of various cancer patients, and inhibitors which inhibit the activity of fusion gene products may be effective for cancer treatment in such fusion gene-expressing patients. The patients who should be treated with inhibitors can be selected from cancer patients by determining whether a fusion gene(s) is(are) expressed in cells, tissues, or body fluids derived from the cancer patients in accordance with the method of the present invention. Therefore, the method of the present invention is especially useful in the field of cancer therapy.

### BRIEF DESCRIPTION OF THE DRAWINGS

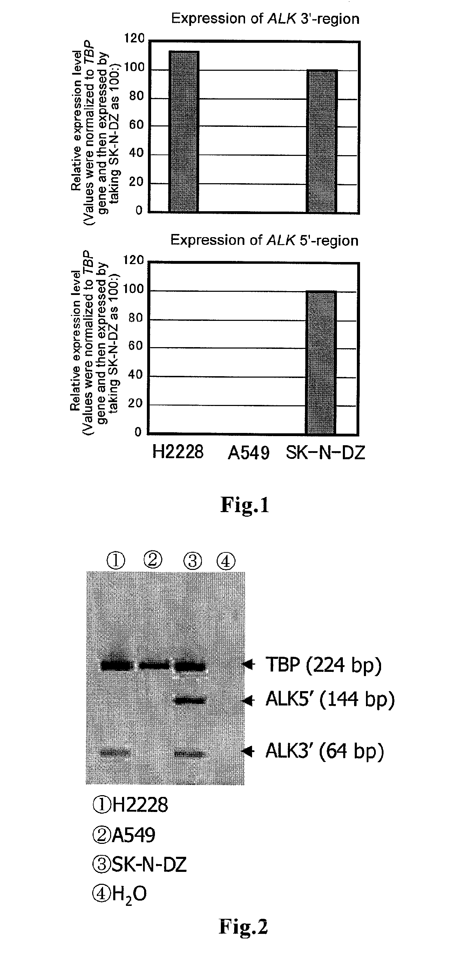

Fig. 1 shows the results of the measurement of the 5'-region and the 3'-region of *ALK* gene on RNA samples extracted from H2228 cells (*EML4-ALK* fusion gene positive, *ALK* gene negative), A549 cells *(EML4-ALK* fusion gene negative, *ALK* gene negative) and SK-N-DZ cells (*EML4-ALK* fusion gene negative, *ALK* gene positive) in Example 1.
Fig. 2 shows the results of the electrophoresis of the amplification products obtained by simultaneous amplification of the 5'-region and the 3'-region of *ALK* gene and the internal control gene *TBP* from RNA samples extracted from H2228 cells, A549 cells and SK-N-DZ cells in Example 2.

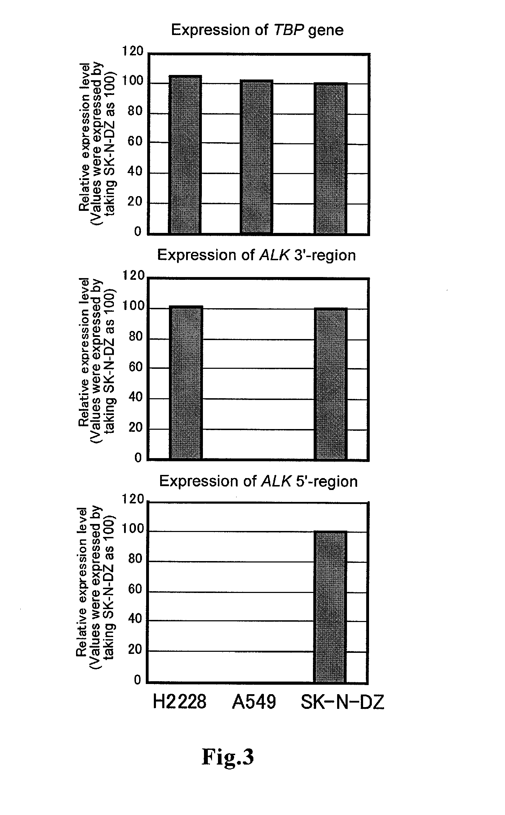

Fig. 3 shows the results of the measurement of the amplification products obtained by simultaneous amplification of the 5'-region and the 3'-region of *ALK* gene and the internal control gene *TBP* from RNA samples extracted from H2228 cells, A549 cells and SK-N-DZ cells using fluorescent probes in Example 3.

### MODE FOR CARRYING OUT THE INVENTION

The fusion gene(s) to be measured in the present invention is(are) a gene generated by fusion between a part of a certain gene and a part of another gene as a result of a translocation (including inversion) within a chromosome or between chromosomes. It is known that as a result of a translocation a gene which is not expressed in a certain tissue under normal circumstances may be fused to a promoter region of another gene which can be expressed in the said certain tissue, thereby being expressed in the said certain tissue to cause various diseases including cancer. Most of the known fusion genes have been identified as an abnormal gene involved in various diseases. Also in the case of the fusion gene(s) to be measured in the present invention, one of the component genes of the fusion gene(s) is usually a gene, the whole of which is not naturally expressed in a normal tissue but a portion of which is allowed to be expressed in the said tissue by gene fusion, and the 5'-region and the 3'-region of the said one of the component genes is measured. However, the requirements of the fusion gene(s) are not restricted by the descriptions above, since whether and how much a fusion gene(s) is(are) expressed can be determined based on the ratio of the expression levels of the 5'-region and the 3'-region as described hereinafter even if the full-length of the component gene is expressed in a normal tissue.

Preferred examples of the fusion gene include those formed by fusion between a gene such as *ALK* gene, *ABL* gene, retinoic acid receptor gene, *SSX* gene, *AML1* gene or the like and another gene. For example, *ALK* gene is a gene encoding receptor tyrosine kinase. Various fusion genes formed by fusion between the 3'-region of *ALK* comprising its kinase domain and the 5'-region of another gene are known, and they are known to be oncogenic (see, e.g. Non-patent Document 5). However, the scope of the present invention is not restricted to these specific examples, and not only fusion genes involved in various diseases such as cancer but also various naturally-occurring fusion genes not involved in any disease are included therein.

*ALK* gene (SEQ ID NO: 1, GenBank NM_004304) is located on human chromosome 2 and consists of 29 exons. The kinase domain spans 4256nt to 5083nt (i.e. the 4256th to the 5083rd bases). The exon regions of *ALK* gene shown in SEQ ID NO: 1 are shown in Table 1 below. As a fusion gene between *ALK* gene and another arbitrary gene (this fusion gene is hereinafter also referred to as *"ALK* fusion gene" for short), fusion genes between *ALK* and various fusion partners are known, as described in Non-patent Document 5. Examples of the known *ALK* fusion gene are shown in SEQ ID NOs: 9 to 18 in SEQUENCE LISTING. SEQ ID NOs: 9 to 17 are the base sequences of *EML4-ALK* fusion genes, and SEQ ID NO: 18 is the base sequence of *KIF5B-ALK* fusion gene. These *ALK* fusion genes are generated by fusion between the region of exons 20 to 29 of *ALK* gene in which the kinase domain is included and a 5'-region of another gene in which promoter region is included, and their expression is observed in some non-small-cell lung cancer patients. As an *ALK* fusion gene, those in which *ALK* is fused to another gene at the 5'-end of exon 20 (SEQ ID NOs: 9 to 12, 14, 18), those in which *ALK* is fused together with several tens of bp of the neighboring intron region (SEQ ID NOs: 15, 16), and those in which several tens to a hundred and several tens of bp of the 5'-end region of exon 20 are lost (SEQ ID NOs: 13, 17) are known. As for the genes listed above as preferred examples besides *ALK* gene, their sequences are known, being registered in databases such as GenBank, and examples of their fusion genes are also known. Therefore, those skilled in the art can carry out the method of the present invention on such fusion genes, in the same manner as on *ALK* fusion genes.

**Table 1**

| | location (nt) | | | location (nt) |
|---|---|---|---|---|
| exon 1 | 1 - 1574 | | exon 16 | 3540 - 3722 |
| exon 2 | 1575 - 1694 | | exon 17 | 3723 - 3821 |
| exon 3 | 1695 - 1859 | | exon 18 | 3822 - 3974 |
| exon 4 | 1860 - 2061 | | exon 19 | 3975 - 4079 |
| exon 5 | 2062 - 2189 | | exon 20 | 4080 - 4266 |
| exon 6 | 2190 - 2321 | | exon 21 | 4267 - 4357 |
| exon 7 | 2322 - 2453 | | exon 22 | 4358 - 4422 |
| exon 8 | 2454 - 2554 | | exon 23 | 4423 - 4552 |
| exon 9 | 2555 - 2724 | | exon 24 | 4553 - 4650 |
| exon 10 | 2725 - 2819 | | exon 25 | 4651 - 4743 |
| exon 11 | 2820 - 2948 | | exon 26 | 4744 - 4845 |
| exon 12 | 2949 - 3111 | | exon 27 | 4846 - 4980 |
| exon 13 | 3112-3262 | | exon 28 | 4981-5071 |
| exon 14 | 3263 - 3394 | | exon 29 | 5072 - 6220 |
| exon 15 | 3395 - 3539 | | | |

In the method of the present invention, one of the component genes which constitute fusion genes is focused on, and the expressions of the two region, i.e., a 5'-region of the gene which is upstream of the fusion point and a 3'-region of the gene which is downstream of the fusion point, are measured. In the present specification, one of the component genes whose 5'-region and 3'-region are to be measured may also be referred to as "target gene" for convenience. The term "measurement" includes detection, quantification and semi-quantification.

As used herein, "fusion point" means the boundary between the region lost by fusion and the region contained in a fusion gene. Focusing on a fusion gene, "fusion point" may also be understood as the boundary at which two different genes are fused. For example, in the case of *ALK* fusion genes shown in SEQ ID NOs: 9 to 12, 14 and 18, the 5'-end of exon 20 of *ALK* gene (i.e. 4080nt in SEQ ID NO: 1) is the fusion point.

The 5'-region and the 3'-region to be measured may be selected from a region upstream of and a region downstream of the fusion point, respectively. It should be understood that, as exemplified by known ALK fusion genes, the fusion point is not necessarily restricted to the just one point in a certain component gene, and that the position of the fusion point may be different among fusion gene variants composed of the same combination of component genes by several tens of bases or more. Therefore, it is desirable to select the 5'-region to be measured from a region upstream of the known fusion point located nearest to the 5'-end. Similarly, it is desirable to select the 3'-region to be measured from a region downstream of the known fusion point located nearest to the 3'-end. Furthermore, in view of covering unknown, not specifically-identified variants, it is preferred to select regions to be measured from a region at a sufficient distance of, e.g., about 200 bases, preferably 500 bases or more, from the most upstream fusion point or the most downstream fusion point.

For example, as described above, most of the known *ALK* fusion genes are those in which exon 20 and its downstream region of *ALK* gene are fused to a fusion partner. Therefore, when measuring the *ALK* fusion gene(s), the 5'-region may be selected from a region *of ALK* exon 19 and its upstream region, and the 3'-region may be selected from a region of *ALK* exon 20 and its downstream region. In addition, because an *ALK* fusion gene in which up to 49 bp in the 5'-end region of exon 20 are lost is known (SEQ ID NO: 13), it is desirable to select the 3'-region from a region downstream of the fusion point of this variant, i.e., 4129nt (which is indicated based on SEQ ID NO: 1; the position of the fusion point is indicated hereinafter in the same manner). Specifically, the 5'-region is preferably selected from a region within 1nt to 4079nt of *ALK* gene (SEQ ID NO: 1), and the 3'-region is preferably selected from a region within 4129nt to 6222nt thereof. Furthermore, there is a possibility that unknown *ALK* fusion genes in which more bases in the 5'-end region of exon 20 are lost may be present. Therefore, in view of detecting *ALK* fusion genes comprehensively, the 3'-region is preferably a region at a sufficient distance, e.g., about 200 bp or more, preferably about 500 bp or more, from the fusion point of 4129nt, which is the known fusion point located nearest to the 3'-end. It is also preferred that the 3'-region be selected from the kinase domain of *ALK* gene. For instance, in the Examples described below, the expression of a region of 2486nt to 2629nt of SEQ ID NO: 1 is measured as the 5'-region, and the expression of a region of 4801nt to 4865nt or 4775nt to 4939nt of SEQ ID NO: 1 is measured as the 3'-region. However, the present invention is not restricted to such a specific example.

The sample used in the present invention is a protein sample or a nucleic acid sample separated from a living body, preferably a nucleic acid sample. In particular, an RNA sample including a total RNA sample extracted from a sample such as cells, tissues, blood or the like collected from a living body is preferred. An RNA sample may be used in the form of cDNA reverse transcribed therefrom. RNA extraction and cDNA synthesis *per se* are well-known conventional methods, and may be easily carried out using commercially available kits. Random hexamers are preferably used as a primer for reverse transcription reaction.

Although measurement of expressions of the 5'-region and the 3'-region may be carried out on a protein sample by immunoassay using antibodies which specifically recognize the 5'-region and the 3'-region, respectively, in the present invention the measurement is preferably carried out by measuring the 5'-region and the 3'-region of mRNA transcribed from the target gene or cDNA synthesized from mRNA. Examples of the method for measurement of mRNA or cDNA include a nucleic acid amplification method in which primers are used or a hybridization method in which probes are used. Examples of the nucleic acid amplification method include RT-PCR, real-time PCR, NASBA and the like. Examples of the hybridization method include Northern blotting, *in situ* hybridization, array analysis in which immobilized probes are used, and the like. These methods *per se* are well-known conventional methods, and any of such methods may be used in the present invention. Immunoassay *per se,* and a method for producing an antibody which specifically recognizes a desired region are also well-known conventional methods.

As primers and probes, polynucleotides which specifically hybridize with the 5'-region and the 3'-region to be measured, respectively, are used. The term "specifically hybridize" used herein means that a certain polynucleotide hybridizes only with the mRNA or cDNA to be measured and does not substantially hybridize with the other nucleic acids under ordinary hybridization conditions. The term "ordinary hybridization condition" refers to a condition used for annealing in the ordinary PCR or the ordinary detection with probes. For example, in the case of PCR with Taq polymerase, the term refers to a reaction condition at an appropriate annealing temperature of about 54°C to 60°C using a common buffer such as one containing 50 mM KCl, 10 mM Tris-HCl (pH 8.3 to 9.0) and 1.5 mM MgCl₂. In the case of Northern hybridization, the term refers to a reaction condition at an appropriate hybridization temperature of 42°C to 65°C using a common hybridization solution such as one containing 5 x SSPE, 50% formamide, 5 x Denhardt's solution and 0.1 to 0.5% SDS. It should be noted, however, that the appropriate annealing temperature and hybridization temperature are not restricted to those exemplified above, and may be determined based on the Tm of the polynucleotide used as a primer or a probe and on the empirical rules. Those skilled in the art can easily determine the appropriate temperature. The term "does not substantially hybridize" means that a hybridization does not occur at all or, even if it occurs, the degree of the hybridization with regions other than the target region is drastically lower than that of the hybridization with the target region so that the hybridization with other regions can be relatively ignored. Those skilled in the art can appropriately design and prepare such polynucleotides, referring to known sequence information. The sequence information can be easily obtained from databases such as GenBank. For example, the base sequence of *ALK* gene has been registered under the accession No. NM_004304 in GenBank, which sequence is also shown in SEQ ID NO: 1 of the SEQUENCE LISTING of the present application.

Specific examples of the measurement method by RT-PCR or real-time PCR include those described in the following Examples. More particularly, cDNA is prepared from each RNA sample extracted from various cells using random hexamers, and then PCR is carried out using the prepared cDNA as a template and using primer sets which specifically amplify the 5'-region and the 3'-region, respectively, of the target gene such as *ALK* gene, so that expressions of the respective regions of the target gene can be measured. As for real-time PCR, detection technique using fluorescently-labeled probe is also known, by which a step of electrophoresis of amplification products can be omitted so that detection can be carry out more simply and more rapidly. In the present invention, fluorescently-labeled probes which specifically hybridize with the fragments amplified from the respective regions may be prepared and used. The amplified fragments can be measured distinctively when the 5'-region and the 3'-region are respectively labeled with fluorescent labels whose wavelengths are different from each other. Those skilled in the art can easily prepare such fluorescently-labeled probes, and a specific example of the probe preparation is described in the following Examples. The term "specifically amplify" as used herein means that only the target region is amplified, or although any regions other than the target region are also amplified, the amount of the amplification thereof is drastically lower than that of the amplification of the target region so that the amplification of other regions can be relatively ignored. A set of primers which specifically hybridize with a region within the 5'-region is a primer set which specifically amplifies the 5'-region. The amplification size may be appropriately selected by those skilled in the art, and is usually not more than 1000 bp, preferably about 30 bp to 500 bp, from the viewpoint of shortening the amplification time, convenience of electrophoretic separation, and so on. For example, in the case where the target gene is *ALK* gene, specific examples of the primer set which specifically amplifies the 5'-region include, but not limited to, the primer set shown in SEQ ID NOs: 3 and 4; and specific examples of the primer set which specifically amplifies the 3'-region include, but not limited to, the primer set shown in SEQ ID NOs: 5 and 6, and the primer set shown in SEQ ID NOs: 20 and 21. Specific examples of the fluorescently-labeled probe include, but not limited to, the *ALK* 5'-region specific probe composed of the base sequence shown in SEQ ID NO: 19, which can be used in combination with the primer set shown in SEQ ID NOs: 3 and 4; and the *ALK* 3'-region specific probe composed of the base sequence shown in SEQ ID NO: 22, which can be used in combination with the primer set shown in SEQ ID NOs: 20 and 21.

In the nucleic acid amplification method, one or a plurality of internal control genes may be measured. As an internal control gene, *GAPDH* gene, *ACTB* gene, *HPRT1* gene, *HMBS* gene, *TBP* gene and the like are well known, and these genes may also be used in the present invention. Fluorescently-labeled probes may also be prepared and used for internal control genes. In the following Examples, *TBP* gene is used as an internal control, and the base sequences of the primer set for *TBP* gene are shown in SEQ ID NOs: 7 and 8 or SEQ ID NOs: 23 and 24, but not limited thereto. Specific examples of the *TBP* gene-specific probe include a probe composed of the base sequence shown in SEQ ID NO: 25, which can be used in combination with the primer set shown in SEQ ID NOs: 23 and 24, but not limited thereto.

After measuring expressions of the 5'-region and the 3'-region, the expressions of the respective regions are compared with each other. In the case where the measurement is carried out by real-time PCR, the expression levels of the regions of the target gene may be normalized to the expression level of the internal control gene and then compared with each other. In the case where amplification products are electrophoresed, the presence or signal intensities of bands may be compared with each other. If the full-length target gene is expressed in the body tissue or cells from which the sample derived, the expressions of both the 5'-region and the 3'-region are observed. If only the fusion gene(s) is(are) expressed therein, the expression of the 5'-region is not observed, and only the expression of the 3'-region is observed. If neither the full length target gene nor the fusion gene(s) is(are) expressed therein, neither the expression of the 5'-region nor the expression of the 3'-region is observed. If both the full length target gene and the fusion gene(s) are expressed, the expressions of both the 5'-region and the 3'-region are observed, and in addition, the expression level of the 3'-region is higher when compared to the case where only the full length target gene is expressed. In such a case, the expression of the fusion gene(s) may be confirmed in more detail by, for example, concurrently carrying out the measurement of a control sample prepared from a tissue or cells in which the full length target gene is expressed but no fusion gene is expressed; determining the ratio of the expression levels of the 5'-region and the 3'-region in the control sample; and then comparing the ratio of the expression levels in the test sample with the ratio in the control sample. Those skilled in the art may easily prepare such control samples referring to known information. For example, in the case of *ALK* gene, examples of the known cell lines in which full length *ALK* gene is expressed and none of the *ALK* fusion genes is expressed include SK-N-DZ cells and the like. Thus, the expression of the fusion gene(s) can be measured based on whether the 5'-region and the 3'-region of the target gene are expressed or not or based on the ratio of the expression levels of the two regions.

For example, it is known that *ALK* fusion gene is expressed in lung cancer cells of some non-small-cell lung cancer patients although the full length *ALK* gene is not expressed in human lung. Therefore, when the present invention is carried out on RNA samples extracted from lung biopsies obtained from lung cancer patients, the expression of the fusion gene(s) can be detected based on whether the 5'-region is expressed or not. That is, in this case, if the expression of the 3'-region is observed and the expression of the 5'-region is not observed in a certain patient, then it can be determined that the *ALK* fusion gene(s) is(are) present and expressed in the patient. The *ALK* fusion gene(s) can also be detected based on whether the 5'-region and the 3'-region are expressed or not or based on the ratio of the expressions thereof when the invention is carried out on RNA samples extracted from blood samples (whole blood, serum, or plasma). *ALK* inhibitors are effective for treatment of cancer in lung cancer patients with *ALK* fusion gene expression, and the method of the present invention makes it possible to select patients who should be treated with *ALK* inhibitors. *ALK* fusion genes are known to be involved in not only lung cancer but also other various cancers, and *ALK* inhibitors are also effective for treatment of various cancers besides lung cancer. Therefore, the method of the present invention is also useful for cancers as well as lung cancer. Moreover, in addition to *ALK* fusion genes, there are other genes known to be involved in cancers, and it is known that inhibitors are also effective for treatment of such cancers. Therefore, the method of the present invention is also useful for cancers in which fusion genes other than *ALK* fusion genes are involved.

The two primer sets composed of the primer set for amplification of the *ALK* 5'-region (SEQ ID NOs: 3, 4) and the primer set for amplification of the *ALK 3'-*region (SEQ ID NOs: 5, 6 or SEQ ID NOs: 20, 21) used in the Examples described below are especially useful as a reagent for detection of an *ALK* fusion gene(s). The reagent may consist only of primers, or may contain various additives useful for stabilization of primers and the like. The reagent may further contain a primer set(s) for internal control. Moreover, the reagent may further contain a fluorescently-labeled probe(s) for real-time PCR. Requirements for the primer set and the fluorescently-labeled probe are as described above. In particular, a combination of the two primer sets described above and the primer set for internal control shown in SEQ ID NOs: 7, 8 or SEQ ID NOs: 23, 24 is a preferred combination established by the present inventor as a combination which can attain simultaneous amplification while reducing non-specific amplification through examination of more than 50 kinds of primer sets. This combination of the three sets makes it especially simple to detect the presence of the *ALK* fusion gene(s) (see, Examples below).

### EXAMPLES

The present invention will now be described more concretely by way of an example thereof. However, the present invention is not restricted to the Examples below.

### Example 1: Preparation and Analysis of RNA Samples 1

H2228 cells (human lung cancer-derived cell line, ATCC), A549 cells (human lung cancer-derived cell line, ATCC), and SK-N-DZ cells (human neuroblastoma-derived cell line, ECACC) were seeded in a culture vessel of 6-well culture plate at a cell density of 20000 cells/cm², and cultured for 2 days to obtain samples. RNA extraction from the samples was carried out using a commercially available RNA extraction kit (Rneasy mini kit, Quiagen) in accordance with the manual to obtain RNA samples. The extracted RNA samples were reverse transcribed into DNA using RT primer (random hexamers, Invitrogen) and Super Script II reverse transcriptase (produced by Invitrogen), thereby obtaining cDNA samples.

The 5'-region and the 3'-region of *ALK* gene and *TBP* gene as an internal control gene which were contained in the cDNA samples were measured by real-time PCR using SYBR Green Realtime PCR Master Mix -Plus- (produced by TOYOBO). The primers used are shown below. The reaction conditions were as follows: thermal denaturation at 96°C for 120 sec, followed by cycle reaction of 96°C for 10 sec, annealing at 60°C for 30 sec, extension at 72°C for 30 sec; the cycle number was appropriately determined by monitoring real-time PCR. The primers used are shown below. As for the composition of the reaction solution, each primer was used at a concentration of 0.8 µM, and buffer etc. attached to the kit were used in accordance with the attached instructions.
*ALK* 5'-region:
   forward (cccgcttctgaaagtgctac, SEQ ID NO: 3), reverse (cccggttttgttctccacta, SEQ ID NO: 4)
*ALK* 3'-region:
   forward (agaggccttcatggaaggaa, SEQ ID NO: 5), reverse (atagcagcactccaaaggac, SEQ ID NO: 6)
*TBP* gene:
   forward (ccaaggaattgaggaagttgc, SEQ ID NO: 7), reverse (gtgccataaggcatcattgg, SEQ ID NO: 8)

The results are shown in Fig. 1. The expression levels of *ALK* gene normalized to the *TBP* gene expression level were expressed as relative values taking the expression levels in SK-N-DZ cells as 100%. The presence of the *ALK*5*'*-region and the *ALK*3*'*-region was confirmed in the cDNA sample prepared from SK-N-DZ cells, indicating that only the normal, unfused *ALK* gene was present. In the sample prepared from H2228 cells, only the *ALK* 3'-region was confirmed to be present, indicating that the *ALK* 5'-region was not expressed and that only the fused *ALK* 3*'-*region was expressed. These results indicate that the presence of the *EML4-ALK* fusion gene(s) can be detected simply by measuring the presence of the 5'-region and the 3'-region of *ALK* gene.

### Example 2: Preparation and Analysis of RNA Samples 2

cDNA samples were prepared from each cells in the same manner as in Example 1. The 5'-region and the 3'-region of *ALK* gene and *TBP* gene as an internal control gene which were contained in the cDNA samples were amplified by multiplex PCR using TITANIUM Taq DNA polymerase (produced by Takara Bio Inc.), and electrophoresis was carried out. The same primers as those used in Example 1 were used. PCR conditions were as follows: thermal denaturation at 96°C for 120 sec, followed by 35 cycles of 96°C for 15 sec and 68°C for 60 sec. As for the composition of the reaction solution, each primer was used at a concentration of 0.8 µM, and buffer etc. attached to the kit were used in accordance with the attached instructions.

The results are shown in Fig. 2. The amplification of the *ALK* 5'-region and the *ALK* 3'-region was confirmed in the cDNA sample prepared from SK-N-DZ cells, whereas, the amplification of only the *ALK* 3'-region was confirmed in H2228 cells. The amplification of the internal control *TBP* gene was confirmed in all the cDNA samples. These results indicate that the presence of the *EML4-ALK* fusion gene(s) can be detected simply by measuring the presence of the 5'-region and the 3'-region of *ALK* gene and an internal control gene.

### Example 3:

RNA samples were obtained from each cells in the same manner in Example 1. The 5'-region and the 3'-region of *ALK* gene and *TBP* gene as an internal control gene which were contained in the RNA samples were measured by One-Step real-time PCR using TITANIUM One-Step RT-PCR Kit (produced by Takara Bio Inc.). The below-described primers and probes for specific detection of the respective amplification products were used. The probes were labeled at the 3'-end with any one of three fluorescent substances whose wavelength was different from one another, which makes it possible to measure the amount of the amplification products by fluorescence quenching caused by a guanine base as known in the art. The reaction conditions were as follows: 50°C for 1 hour, and thereafter thermal denaturation at 96°C for 120 sec, followed by cycle reaction of 96°C for 10 sec, annealing at 60°C for 30 sec, extension at 72°C for 30 see; the cycle number was appropriately determined by monitoring real-time PCR. As for the composition of the reaction solution, each primer was used at a concentration of 0.8 µM, each probe was used at a concentration of 0.1 µM, and buffer etc. attached to the kit were used in accordance with the attached instructions.
*ALK* 5'-region :
   forward (cccgcttctgaaagtgctac, SEQ ID NO: 3), reverse (cccggttttgttctccacta, SEQ ID NO: 4), probe (tctccatgtgagctccgaatgtcc, the cytosine base at 3'-end was labeled with TAMRA, SEQ ID NO: 19)
*ALK* 3'-region :
   forward (atgctgccagttaagtggatg, SEQ ID NO: 20), reverse (actggtgacaaactccagaac, SEQ ID NO: 21), probe (tatgccataccccagcaaaagcaac, the cytosine base at 3'-end was labeled with BODIPY FL, SEQ ID NO: 22)
TBP gene :
   forward (cttggcgtgtgaagataacc, SEQ ID NO: 23), reverse (tgctgcctttgttgctcttc, SEQ ID NO: 24), probe (ccttacgctcagggcttggcctcc, the cytosine base at 3'-end was labeled with 5-CR6G, SEQ ID NO: 25)

The results are shown in Fig. 3. The expression levels of *TBP* gene, *ALK 5'-*region and *ALK* 3'-region were expressed as relative values taking the expression levels in SK-N-DZ cells as 100%. The amplification of the *ALK* 5'-region and the *ALK* 3'-region was observed in RNA sample derived from SK-N-DZ cells, whereas, the amplification of only the *ALK* 3'-region was observed in H2228 cells. The amplification of internal control *TBP* gene was observed in all the RNA samples. These results indicate that the presence of the *EML4-ALK* fusion gene(s) can be detected directly by measuring the presence of the 5'-region and the 3'-region of *ALK* gene and an internal control gene.

## Claims

1. A method for measuring a fusion gene(s), which is applied to a sample separated from a living body, said method comprising: measuring expressions of a 5'-region and a 3'-region of one of the component genes of the fusion gene(s) which may be present in the living body, wherein said 5'-region is upstream of and said 3'-region is downstream of a fusion point in said one of the component genes; and comparing the expression of the 5'-region with the expression of the 3'-region.

2. The method according to claim 1, wherein said one of the component genes is *ALK* gene.

3. The method according to claim 2, wherein said 5'-region is any region within the region of exons 1 to 19 of *ALK* gene, and said 3'-region is any region within the region of exons 20 to 29.

4. The method according to claim 3, wherein said 5'-region is any region within the 1nt to 4079nt region of the *ALK* gene sequence shown in SEQ ID NO: 1, and said 3'-region is any region within the 4129nt to 6222nt region of said *ALK* gene sequence.

5. The method according to any one of claims 1 to 4, wherein said sample is an RNA sample, and the expression measurement is carried out by measuring the 5'-region and the 3'-region of mRNA transcribed from *ALK* gene or cDNA synthesized from said mRNA.

6. The method according to claim 5, which method is carried out by RT-PCR using a primer set which specifically amplifies the 5'-region and a primer set which specifically amplifies the 3'-region.

7. The method according to claim 6, wherein a 5'-region detection primer set composed of the base sequences shown in SEQ ID NOs: 3 and 4 of the SEQUENCE LISTING, respectively, and a 3'-region detection primer set composed of the base sequences shown in SEQ ID NOs: 5 and 6, respectively, are used.

8. The method according to claim 7, wherein an internal control primer set composed of the base sequences shown in SEQ ID NOs: 7 and 8, respectively, is further used.

9. The method according to any one of claims 2 to 8, wherein said living body is a cancer patient.

10. The method according to claim 9, wherein said living body is a lung cancer patient.

11. A reagent for detection of a fusion gene(s) formed by fusion with *ALK* gene, said reagent comprising a 5'-region detection primer set composed of the base sequences shown in SEQ ID NOs: 3 and 4 of the SEQUENCE LISTING, respectively, and a 3'-region detection primer set composed of the base sequences shown in SEQ ID NOs: 5 and 6, respectively.

12. A reagent for detection of a fusion gene(s) formed by fusion with *ALK* gene, said reagent comprising a 5'-region detection primer set composed of the base sequences shown in SEQ ID NOs: 3 and 4 of the SEQUENCE LISTING, respectively, and a 3'-region detection primer set composed of the base sequences shown in SEQ ID NOs: 20 and 21, respectively.

13. The reagent according to claim 11, further comprising an internal control primer set composed of the base sequences shown in SEQ ID NOs: 7 and 8, respectively.

14. The reagent according to claim 12, further comprising an internal control primer set composed of the base sequences shown in SEQ ID NOs: 23 and 24, respectively.
